Europäisches Patentamt

European Patent Office

Office européen des brevets

(19)

(11) Publication number: **0 308 983**

**A2**

(12) **EUROPEAN PATENT APPLICATION**

(21) Application number: 88115806.7

(51) Int. Cl.⁴: **A61K 49/00**

(22) Date of filing: 26.09.88

(30) Priority: 25.09.87 US 101327

(43) Date of publication of application:
**29.03.89 Bulletin 89/13**

(84) Designated Contracting States:
**AT BE CH DE ES FR GB GR IT LI LU NL SE**

(71) Applicant: **SALUTAR, INC.**
**428 Oakmead Parkway**
**Sunnyvale California 94086(US)**

(72) Inventor: Quay, Steven C.
**27863 Moody Road**
**Los Altos Hills California 94022(US)**

(74) Representative: Barz, Peter, Dr. et al
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) NMR imaging with Mn(II) coordination compositions.

(57) Enhanced $T_1$ and $T_2$ relaxation in NMRI is obtained with solutions of non-chelate coordination compounds of the formula:

$$[Mn(II)(H_2O)_m A1_n A2_o A3_p A4_q]^{+a}(Y, Z_r)^{-a}$$

In the formula,
A1, A2, A3 and A4 are the same of different amino-substituted carboxylic acid groups having from 2 to 18 carbons;
Y and Z are each the same or a different anion of a pharmaceutically acceptable inorganic acid or an organic carboxylic acid having from 2 to 18 carbons;
a is valence of the ions;
m, n, o, p, and q are each 1 or 0, $(m + n + o + p + q) = 4$, and $(n + o + p + q)$ is preferably from 1 to 3; and
r is 1, and if Y is a multivalent anion, r is 0 or 1.
The solution has a pH within the range of from 4 to 9.5, preferably from 5 to 7, and optimally from 5 to 6.5, and for parenteral administration, a coordination compound concentration of from 0.5 to 4.0 wt%. Preferably, n is from 1 to 3, optimally from 2-4, and most optimally from 2 to 3, and Y and Z are monovalent. For example Y is preferably an anion of an aliphatic carboxylic acid having from 2 to 6 carbons such as acetic, propionic, n-butyric acid, isobutyric acid, valeric acid, isovaleric acid, pivalic acid or gluconic acid, and Z is an inorganic anion such as chloride or sulfate. A1, A2, A3 and A4 are preferably α-amino acids having from 2 to 6 carbons such as glycine.

EP 0 308 983 A2

# NMR IMAGING WITH NOVEL Mn(II) COORDINATION COMPOSITIONS

This invention relates to novel nuclear magnetic resonance (NMR) imaging compositions containing manganese(II) coordination compounds and their use in nuclear magnetic resonance imaging (NMRI). In particular, this invention is directed to manganese(II) coordination complexes with water-soluble amino acids, to NMRI imaging compositions containing these complexes, and to their use in NMRI imaging.

Manganese(II) was early identified as an optimuM NMRI contrast agent. With five unpaired electrons, $Mn^{+2}$ in solution is coordinated with six molecules, forming the $[manganese(II) \cdot (H_2O)_4]^{+2}$ cation. It was also naturally present in the body. Use of $[manganese(II) \cdot (H_2O)_4]^{+2}(Cl_2)^{-2}$ solutions to increase contrast and delineate myocardium areas affected by infarcts in dogs for determining infarct size was reported by Goldman, M. et al, Circulation. 66:1012-1016 (1982). However, the free ion is toxic and has a delayed clearance. Kang, Y. et al, Invest.Radiol. 19:399-407 (1984), studying the $R_2/R_1$ ratio of manganese in heart, liver, spleen and kidney, reported that manganese in hart, liver, spleen and kidney, reported that manganese provided unusually beneficial relaxation characteristics at low concentrations, and a linearity between relaxation rates and the injected dose was observed over the entire range up to 0.09 mmole/kg. Mendonca-dias, M. et al, Seminars in Nuclear Medicine. 13:364-376 (1983) also reported that doses of manganese injected into rats in excess of 0.1 mmole/kg of body weight were often lethal, and the effect of manganese is effectively toxicity limited.

Strich, G. et al, J.Urology. 136:1216-1219 (1984) found that striking $T_1$ relaxations were achieved with doses of only 0.03 mmole/kg of $[manganese(II) \cdot (H_2O)_4]^{+2}(Cl_2)^{-2}$, but use of the compound was limited by toxicity. Wolf, G. et al, A.J.R. 141:193-197 (1983) reported that both PR intervals and QaTc intervals for both dogs and rabbits were prolonged by manganese at all doses (0.01, 0.02 and 0.1 mmole/kg), and when administered at doses of 0.2 mmole/kg to 4 dogs, three experienced ventricular fibrillation and the fourth developed severe hypotension. One minute after injection, all animals experienced a dose-dependent drop in arterial pressure. Yanaga, T. et al, Am.J.Physiol. 217:12801286 (1969) studied the mechanism of manganese action on cardiac muscle, finding evidence that the manganese not only altered the transmembrane potential but also acted on excitation-contraction coupling to produce a decrease in tension. Sanborn, W. et al, Circulation Research. 43:178-187 (1978) investigated the manganese action on excitation-contraction coupling, finding the uncoupling action was exerted in the interstitium. In studies of Gd-DTPA, no measurable changes in arterial blood pressure or EKG intervals were observed by Wolf, G. et al, Invest.Radiology. 19:324-328 (1984), noting that this contrasted with earlier studies with $MnCl_2$. Carr, E. Clin.Pharmacol.Ther. 35:131-140 (1984) reported that paramagnetic effects of ions such as manganese are, at first glance, unpromising because their toxicity is likely to preclude their administration in amounts large enough to be useful in NMR.

Mendonca-dias, M. et al, (supra p 373) reported that although doses of 0.1 mmol/kg $[manganese(II) \cdot (H_2O)_4]^{+2}(Cl_2)^{-2}$ were almost always lethal for rats, injection of EDTA can block the effect and restore the ECG pattern. Paramagnetic chelates were found to be less toxic than the free ions and were shown to function as effective paramagnetic contrast agents. Carr, D. et al, Clin.Radiology. 36:561-568 (1985) noted that the problem of complexing is that it displaces much of the coordinated water. Coordination of manganese ion with water is important for effective relaxation. Since the coordination number of $Gd^{+3}$ and $Mn^{+2}$ ions exceeds the available ligand sites for EDTA and DTPA, some water can occupy inner coordination sites. Although relaxation effects are reduced by chelating, the effect was greatly outweighed by the increased safety of the complex. Reduction of toxic effects by slow administration of $[manganese(II) \cdot (H_2O)_4]^{+2}(Cl_2)^{-2}$ was reported by Slutsky, R. et al, Radiology. 154:733-735 (1985).

Manganese chelates of DTPA, desferrioxamine B, glucoheptonic acid and phytate were evalutated for NMR image enhancement by Huberty, J. et al, Society of Magnetic Resonance in Medicine, Scientific Programme. pp 175-176, Society of Magnetic Resonance in Medicine (1983).

Chelates of $Mn^{+2}$ with a variety of chelating agents have been investigated. Use of alkylenediamine chelates with a variety of paramagnetic ions including Mn(II) are described in U.S. Patent 4,647,447. Manganese(II) is included in a list of suitable paramagnetic metal ions for use with polysaccharide derivatives of a variety of chelating compounds including EDTA, DTPA and aminoethyl diphosphonate in PCT application publication no. WO85/05554 (Application No. PCT/GB85/00234).

Other studies of NMR manganese chelates have been reported by Frost, et al, J.Am.Chem.Soc. 80:530 (1958), L'Eplathenier, F. et al, J.Am.Chem.Soc. 89:837 (1967), Patch et al, Inorg.Chem. 21:2972-2977 (1982), and U.S. Patent 3,632,637.

Kang, Y. et al, (supra p 406), studying the enhanced effects of binding of paramagnetics with tissues, observed that chelation of the ion will prevent or reduce such enhancements so that greater amounts of the complex would be needed to produce the same effect. He recommended designing a complexing agent which both reduces toxicity and preserves or provides large values of $\epsilon^*$ ($\epsilon^* = \Delta_{solution}/\Delta_{water}$). Schuhmacher, J. et al, *Invest.Radiol*, 20:601-608 (1985) reported that the relaxation rate of $Mn^{+2}$ was decreased when complexed with DTPA, appeared unaffected when complexed with penicillamine (PA) chelates, and was enhanced when added to a chelating solution of iminodiacetic acid substituted bromophthalein.

Shaefer, S. et al, BOOK OF ABSTRACTS, VOLUME 1. Sixth Annual Meeting and Exhibition August 17-21, 1987 of the Society of Magnetic Resonance in Medicine in New York City, p 329 describes a study of NMR imaging with $[manganese(II) \cdot (H_2O)_4]^{+2}(gluconate_2)^{-2}$ with calcium gluconate compositions.

This invention is based on a discovery that non-chelate coordination complexes of amino acids with $Mn^{+2}$ substantially reduce toxicity without the reduction in relaxivity experienced with chelates, yielding hitherto unrealized levels of relaxation.

Clegg, W. et al, *Acta.Cryst.*C43:794-797 (1987) reports studies of glycine-bridged crystal structures wherein the repeating unit is $Mn(glycine)(H_2O)_2Cl_2$. Led, J. *J.Am.Chem.Soc.* 107:6755-6765 (1985) reported paramagnetic relaxation rate studies of the glycine carbon-13 atoms and the ATP phosphorus-31 atoms of the ternary chelated $glycine/Mn^{+2}/ATP^{-4}$ system in water-glycerol at pH 7.4.

Chelates and other organic complexes have been traditionally used to solubilize and stabilize essential metal ions needed for nutrition and medical treatment. U.S. Patent 3,950,372 describes manganese complexes with $\alpha$-amino acids such as methionine and glycine in crystalline form for dietary supplementation. As shown in Example 38 herein, the method of this patent produces chelates giving small relaxivity when compared with the large relaxivity provided by the compounds of this invention.

U.S. Patent 4,167,564 describes complexes of manganese ions and amino acids in a weight ratio of amino acid to manganese ion of 5:1 to 100:1, compositions containing from 20 mg to 1 gm of amino acid and from 1 to 10 mg of Manganese, denoting them as "chelated coordination complexes".

The method of this invention for performing an NMR diagnostic procedure in a patient comprises administering to the patient a physiologically acceptable solution of an NMR image enhancing amount of a non-chelate coordination compound of the formula:

$$[Mn(II)(H_2O)_m A1_n A2_o A3_p A4_q]^{+a}(Y,Z_r)^{-a}$$

In the formula,

A1, A2, A3 and A4 are the same or different amino-substituted carboxylic acid groups having from 2 to 18 carbons;

Y and Z are each the same or a different anion of a pharmaceutically acceptable inorganic acid or an organic carboxylic acid having from 2 to 18 carbons;

a is valence of the ions;

m, n, o, p, and q are each 1 or 0, (m + n + o + p + q) = 4, and (n + o + p + q) is preferably from 1 to 3; and

r is 1, and if Y is a multivalent anion, r is 0 or 1.

The solution has a pH within the range of from 4 to 9.5, preferably from 5 to 7, and optimally from 5 to 6.5, and for parenteral administration, a coordination compound concentration of from 0.5 to 4.0 wt%. Preferably, n is from 1 to 3, optimally from 2-4, and most optimally from 2 to 3, and Y and Z are monovalent. For example Y is preferably an anion of an aliphatic carboxylic acid having from 2 to 10, more preferably from 2 to 8, and optimally from 2 to 6 carbons such as acetic, propionic, n-butyric, isobutyric, valeric, isovaleric, lactic, pivalic or gluconic acid, and Z is an inorganic anion such as chloride or sulfate: A1, A2, A3 and A4 are preferably $\alpha$-amino acids having from 2 to 6 carbons such as glycine.

Pharmaceutically acceptable parenteral solutions of the coordination compound having a pH within the range of from 5 to 7, and a coordination compound concentration of from 0.5 to 4.0 wt% are also included within the scope of this invention. Preferred compositions have a value for "n" in the formula of 1.5 to 4 and optimally from 2 to 3.

In the drawings:

Fig. 1-3 are schematic representations of NMR images of internal organs of a rabbit as a function of time with administration of $[manganese(II) \cdot (H_2O)_2 \cdot (glycine)_2]Cl_2$ in Example 27.

Fig. 4 is a plot of $1/T_1 \times 1000$ values vs time from the data in Table C, Example 28. This shows the extended time for clinical usefulness of $[manganese(II) \cdot (H_2O)_2 \cdot (glycine)_2]Cl_2$ over that obtainable with GdDTPA at the same concentration.

Fig. 5 is a plot of $T_1$ vs time from the data in Table C, Example 28. This shows the improved relaxivity obtained with [manganese(II)•(H_2O)_2•(glycine)_2]Cl_2 over that obtainable with GdDTPA at the same concentration.

Toxicity of paramagnetic metals has been reduced with chelates or by use of insoluble forms of the metal for oral gastrointestinal contrast agents. However, relaxation effects are reduced by both approaches, and the latter approach is not suitable for parenteral dosage forms.

This invention is based on the discovery that soluble non-chelated amino acid coordination compounds of manganese(II) provide the same relaxivity as manganese salts but with a seven-fold improvement in safety. These compounds provide the greatest enhancement of relaxivity in cardiac and liver tissue previously reported, providing values more than ten times lower than the values obtainable with the best chelates, at any dose.

Manganese salts have the highest known relaxivity in vivo but are extremely toxic. Manganese chelates provide greatly improved safety (often lowering toxicity by 50-fold compared with manganese salts) but only at the expense of substantial (often equal) lowering of relaxivity. This would potentially require large doses of chelates, thus cancelling the gains in safety.

The non-chelated compounds of this invention appear to not significantly affect the binding of manganese to body tissues and blood components, and particularly heart and liver tissue, and yet they significantly reduce the toxicity. These compounds are believed to provide a higher level of relaxivity in vivo in certain organs such as the liver and heart than can be achieved with manganese chelates and possibly other (including $Gd^{+3}$) paramagnetic metal compositions.

The coordination compounds useful in this invention can be represented by the formula:

$$[Mn(II)(H_2O)_m A1_n A2_o A3_p A4_q]^{+a} (Y,Z_r)^{-a}$$

In the formula,

A1, A2, A3 and A4 are the same or different amino-substituted carboxylic acid groups having from 2 to 18 carbons;

Y and Z are each the same or a different anion of a pharmaceutically acceptable inorganic acid or an organic carboxylic acid having from 2 to 18 carbons;

a is valence of the ions;

m, n, o, p, and q are each 1 or 0, (m + n + o + p + q) = 4, and (n + o + p + q) is preferably from 1 to 3; and

r is 1, and if Y is a multivalent anion, r is 0 or 1.

Non-chelated coordination complexes of manganese(II) are formed by mixing a soluble manganese(II) salt solution with a solution of the amino acid in the molar ratios desired in the final product.

Suitable manganese(II) salts of inorganic anions are chlorides, bromides, iodides, fluorides, sulfates, phosphates, and preferably chloride and sulfate. Suitable manganese(II) salts of organic anions are water-soluble salts of manganese(II) with carboxylic acids, preferably aliphatic carboxylic acids having from 2 to 16 carbons and optimally from 2 to 6 carbons such as acetic, propionic, n-butyric, isobutyric, valeric, lactic, isovaleric, pivalic or gluconic acid,.

The term "aliphatic carboxylic acid", as used herein, is defined to include alkyl and alkenyl carboxylic acids having up to 18 carbons and alkyl, halo (fluoro, chloro, bromo or iodo) and hydroxy substituted derivatives thereof.

The amino acid can be any physiologically acceptable, water-soluble amino substituted carboxylic acid having from 1 to 16 carbons such as those listed together with literature references describing their origin in the naturally occurring amino acids described in HANDBOOK OF BIOCHEMISTRY, New York: Chemical Rubber Company, pp B3-B54 (1970), the entire contents of which and the references cited therein being hereby incorporated by reference in their entireties. The preferred amino acids are α-amino acids having from 2 to 12 carbons.

The solutions should have a pH which retains the coordination complexes in an unchelated form. In general the pH is within the range of from 4 to 9.5, preferably from 5 to 7 and optimally from 5 to 6.5.

The solution concentration should be sufficiently high to provide the desired image relaxation and image enhancement when administered in the selected form and selected amount. For parenteral administration for imaging the heart, for example, the molar concentration should provide a dosage of manganese(II) ion within the range of from 5 to 50 μmole/kg and preferably within the range of from 10 to 30 μmole/kg. The parenteral dosage of manganese ion is at least 11 mg and preferably from 30 to 460 mg per dose.

The solution concentration can be from 0.5 to 4 wt.% of the manganese(II) coordination compound.

The preferred compositions are formed by mixing the pure manganese(II) salt and amino acid in sterile, deionized water. No other additives are necessary. The compositions can contain usual pharmaceutical acceptable buffers, osmolarity modifiers, antimicrobial preservatives, antioxidants, emulsifying and/or solubilizing agents, solvents, suspending or viscosity increasing agents, wetting and/or solubilizing agents, and other excipients, if desired, as is within the skill of the art. However, any excipients should be carefully chosen so as not to interfere with the stability of the non-chelated manganese(II) coordination compounds.

The manganese(II) coordination compound solutions of this invention can be administered by any of the usual modes, selected to provide the coordination compound to the portion of the body to be imaged by the NMR device. They can be administered rectally, orally or parenterally. The preferred compositions are designed for parenteral administration, for imaging the head, heart, liver and kidney. In particular, the image enhancement of the heart with physiologically acceptable levels of the manganese(II) coordination compounds of this invention are substantially superior to enhancement which can be achieved with safe levels of manganese salts, chelate complexes, and other paramagnetic metals.

This invention is further illustrated by the following specific, but non-limiting examples. Temperatures are given in degrees Centigrade and percents as weight percents unless otherwise specified. Examples which are constructively reduced to practice herein are presented in the present tense, and examples representing laboratory experiments previously reduced to practice are presented in the past tense.

## EXAMPLE 1

$$[Manganese(II) \cdot (H_2O)_2 \cdot (L\text{-}alanine)_2]^{+2}(Cl_2)^{-2}$$

A non-chelate coordination complex solution of $[manganese(II) \cdot (H_2O)_2 \cdot (L\text{-}alanine)_2]^{+2}(Cl_2)^{-2}$ was formed in aqueous media at room temperature. Manganese chloride (Baker analytical grade 99.5% purity, lot #434113 $MnCl_2 \cdot 4H_2O$; MW 197.07; 1.98 gm; 10 mmoles) was dissolved in 100 ml of deionized water to form a 100 mM clear solution. L-alanine (α-aminopropionic acid) (Pierce Chemical Co., Rockford, IL; MW 89.09; 93.2 mg; 1.1 mmoles) was added to 20 parts of deionized water to form a 523 mM solution. 40 Parts by volume of the $MnCl_2$ solution were added to 15 parts of above formed amino acid solution to give, instantaneously, a non-chelate coordination compound solution of $[manganese(II) \cdot (H_2O)_2 \cdot (L\text{-}alanine)_2]^{+2}(Cl_2)^{-2}$

The paramagnetic relaxation properties of $[manganese(II) \cdot (H_2O)_2 \cdot (L\text{-}alanine)_2]^{+2}(Cl_2)^{-2}$ in water were measured at 10 MHz (0.15T) with a spin analyzer (RADX; Houston, TX) at 37° C. After thermal equilibrium, the spin-lattice ($T_1$) and spin-spin ($T_2$) relaxation values were measured in triplicate. The $T_1$ values of a 0.99 mM solution were 104 +/-9 msec (means +/- standard deviation) and the $T_2$ values were 26 +/- 1 msec. These values permitted the calculation of millimolar relaxivity values of 9.81 $mM^{-1}sec^{-1}$ for $T_1$ and 39.3 $mM^{-1}sec^{-1}$ for $T_2$.

The paramagnetic relaxation properties were also determined in human plasma at 37° C. The title compound was added to human plasma at a 94 μM final concentration (the total dilution of the plasma was 10.5%). The plasma remained clear yellow. The $T_1$ value (in triplicate) was 238 +/- 11 msec, the $T_2$ value was 187 +/- 3 msec, the $R_1$ was 44.7 $mM^{-1}sec^{-1}$, and the $R_2$ was 56.9 $mM^{-1}sec^{-1}$. These values were stable based on a repeat measurement (80 minutes later) which indicated $T_1$ changed by 3% and $T_2$ changed by 1%. Thus, the $T_1$ relaxivity of $[manganese(II) \cdot (H_2O)_2 \cdot (L\text{-}alanine)_2]^{+2}(Cl_2)^{-2}$ in plasma is 9.3-fold greater than gadolinium diethylenetriaminepentaacetic acid chelate (GdDTPA). The $T_2$ relaxivity is 11.9-fold greater than GdDTPA in plasma.

## EXAMPLE 2

$$[Manganese(II) \cdot (H_2O)_2 \cdot (L\text{-}leucine)_2]^{+2}(Cl_2)^{-2}$$

A non-chelate coordination compound solution of $[manganese(II) \cdot (H_2O)_2 \cdot (L\text{-}leucine)_2]^{+2}(Cl_2)^{-2}$ was formed in aqueous media at room temperature. Manganese chloride (Baker analytical grade 99.5% purity, lot #434113 $MnCl_2 \cdot 4H_2O$; MW 197.07; 1.98 gm; 10 mmoles) was dissolved in 100 ml of deionized water to form a 100 m$M$ clear solution. $L$-leucine ($\alpha$-aminoisocaproic acid) (Pierce Chemical Co., Rockford, IL; MW 131.18; 20.3 mg; 0.2 mmoles) was added to 200 parts of deionized water to form a 38.7 m$M$ solution. 40 Parts by volume of the $MnCl_2$ solution were added to 207 parts of above formed amino acid solution to give, instantaneously, a non-chelate coordination compound solution of $[manganese(II) \cdot (H_2O)_2 \cdot (L\text{-}leucine)_2]^{+2}(Cl_2)^{-2}$.

The paramagnetic relaxation properties of $[manganese(II) \cdot (H_2O)_2 \cdot (L\text{-}leucine)_2]^{+2}(Cl_2)^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.94 m$M$ solution were 114 +/- 9 msec (mean +/- standard deviation) and the $T_2$ values were 27 +/- 1 msec. These values permitted the calculation of millimolar relaxivity values of 9.33 m$M^{-1}$sec$^{-1}$ for $T_1$ and 39.4 m$M^{-1}$sec$^{-1}$ for $T_2$.

The paramagnetic relaxation properties were also determined in human plasma at 37°C. The title compound was added to human plasma at a 90$\mu M$ final concentration (the total dilution of the plasma was 10.5%). The plasma remained clear yellow. The $T_1$ value (in triplicate) was 243 +/- 13 msec, the $T_2$ value was 192 +/- 0 msec, the $R_1$ was 45.7 m$M^{-1}$sec$^{-1}$, and the $R_2$ was 57.9 m$M^{-1}$sec$^{-1}$. These values were stable based on a repeat measurement (80 minutes later) which indicated $T_1$ changed by 5% and $T_2$ changed by 4%. Thus, the $T_1$ relaxivity of $[manganese(II) \cdot (H_2O)_2 \cdot (L\text{-}leucine)_2]^{+2}(Cl_2)^{-2}$ in plasma is 9.5-fold greater than gadolinium diethylenetriaminepentaacetic acid chelate (GdDTPA). The $T_2$ relaxivity is 12.1-fold greater than GdDTPA in plasma.

## EXAMPLE 3

$$[Manganese(II) \cdot (H_2O)_2 \cdot (glycine)_2]^{+2}(Cl_2)^{-2}$$

A non-chelate coordination compound solution of $[manganese(II) \cdot (H_2O)_2 \cdot (glycine)_2]^{+2}(Cl_2)^{-2}$ was formed in aqueous media at room temperature. Manganese chloride (Baker analytical grade 99.5% purity, lot #434113 $MnCl_2 \cdot 4H_2O$; MW 197.07; 1.98 gm; 10 mmoles) was dissolved in 100 ml of deionized water to form a 100 m$M$ clear solution. Glycine ($\alpha$-aminoacetic acid) (Pierce Chemical Co., Rockford, IL; MW 75.07; 54.2 mg; 0.7 mmoles) was added to 40 parts of deionized water to form a 361 m$M$ solution. 40 Parts by volume of the $MnCl_2$ solution were added to 22 parts of above formed amino acid solution to give, instantaneously, a non-chelate coordination compound solution of $[manganese(II) \cdot (H_2O)_2 \cdot (glycine)_2]^{+2}(Cl_2)^{-2}$.

The paramagnetic relaxation properties of $[manganese(II) \cdot (H_2O)_2 \cdot (glycine)_2]^{+2}(Cl_2)^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.99 m$M$ solution were 109 +/-5 msec (means +/- standard deviation) and the $T_2$ values were 26 +/- 1 msec. These values permitted the calculation of millimolar relaxivity values of 9.27 m$M^{-1}$sec$^{-1}$ for $T_1$ and 38.9 m$M^{-1}$sec$^{-1}$ for $T_2$.

The paramagnetic relaxation properties were also determined in human plasma at 37°C. The title compound was added to human plasma at a 94 $\mu M$ final concentration (the total dilution of the plasma was 10.5%). The plasma remained clear yellow. The $T_1$ value (in triplicate) was 227 +/- 3 msec, the $T_2$ value was 184 +/- 12 msec, the $R_1$ was 46.9 m$M^{-1}$sec$^{-1}$, and the $R_2$ was 57.8 m$M^{-1}$sec$^{-1}$. These values were stable based on a repeat measurement (80 minutes later) which indicated $T_1$ changed by 2% and $T_2$ changed by 2%. Thus, the $T_1$ relaxivity of $[manganese(II) \cdot (H_2O)_2 \cdot (glycine)_2]^{+2}(Cl_2)^{-2}$ in plasma is 9.8-fold greater than gadolinium diethylenetriaminepentaacetic acid chelate (GdDTPA). The $T_2$ relaxivity is 12.0-fold greater than GdDTPA in plasma.

## EXAMPLE 4

$$[Manganese(II) \cdot (H_2O)_2 \cdot (L\text{-}phenylalanine)_2]^{+2}(Cl_2)^{-2}$$

A non-chelate coordination compound solution of [manganese(II) $\cdot$ (H$_2$O)$_2$ $\cdot$ (L-phenylalanine)$_2$]$^{+2}$(Cl$_2$)$^{-2}$ was formed in aqueous media at room temperature. Manganese chloride (Baker analytical grade 99.5% purity, lot #434113 MnCl$_2$ $\cdot$ 4H$_2$O; MW 197.07; 1.98 gm; 10 mmoles) was dissolved in 100 ml of deionized water to form a 100 mM clear solution. L-phenylalanine (Pierce Chemical Co., Rockford, IL; MW 165.19; 33.0 mg; 0.2 mmoles) was added to 120 parts of deionized water to form a 49.9 mM solution. 40 Parts by volume of the MnCl$_2$ solution were added to 160 parts of above formed amino acid solution to give, instantaneously, a non-chelate coordination compound solution of [manganese(II) $\cdot$ (H$_2$O)$_2$ $\cdot$ (L-phenylalanine)$_2$]$^{+2}$(Cl$_2$)$^{-2}$.

The paramagnetic relaxation properties of [manganese(II) $\cdot$ (H$_2$O)$_2$ $\cdot$ (L-phenylalanine)$_2$]$^{+2}$(Cl$_2$)$^{-2}$ in water were measured at 10 MHz (0.15T) with a spin analyzer (RADX; Houston, TX) at 37°C. After thermal equilibrium, the spin-lattice (T$_1$) and spin-spin (T$_2$) relaxation values were measured in triplicate. The T$_1$ values of a 0.95 mM solution were 107 +/- 5 msec (mean +/- standard deviation) and the T$_2$ values were 27 +/- 1 msec. These values permitted the calculation of millimolar relaxivity values of 9.84 mM$^{-1}$sec$^{-1}$ for T$_1$ and 39.0 mM$^{-1}$sec$^{-1}$ for T$_2$.

The paramagnetic relaxation properties were also determined in human plasma at 37°C. The title compound was added to human plasma at a 90μM final concentration (the total dilution of the plasma was 10.5%). The plasma remained clear yellow. The T$_1$ value (in triplicate) was 240 +/- 10 msec, the T$_2$ value was 186 +/- 7 msec, the R$_1$ was 46.3 mM$^{-1}$sec$^{-1}$, and the R$_2$ was 59.7 mM$^{-1}$sec$^{-1}$. These values were stable based on a repeat measurement (80 minutes later) which indicated T$_1$ changed by 2% and T$_2$ changed by 2%. Thus, the T$_1$ relaxivity of [manganese(II) $\cdot$ (H$_2$O)$_2$ $\cdot$ (L-phenylalanine)$_2$]$^{+2}$(Cl$_2$)$^{-2}$ in plasma is 9.65-fold greater than gadolinium diethylenetriaminepentaacetic acid chelate (GdDTPA). The T$_2$ relaxivity is 12.4-fold greater than GdDTPA in plasma.

## EXAMPLE 5

$$[Manganese(II) \cdot (H_2O)_2 \cdot (L\text{-}isoleucine)_2]^{+2}(Cl_2)^{-2}$$

A non-chelate coordination compound solution of [manganese(II) $\cdot$ (H$_2$O)$_2$ $\cdot$ (L-isoleucine)$_2$]$^{+2}$(Cl$_2$)$^{-2}$ was formed in aqueous media at room temperature. Manganese chloride (Baker analytical grade 99.5% purity, lot #434113 MnCl$_2$ $\cdot$ 4H$_2$O; MW 197.07; 1.98 gm; 10 mmoles) was dissolved in 100 ml of deionized water to form a 100 mM clear solution. L-isoleucine (α-amino-γ-methylvaleric acid) (Pierce Chemical Co., Rockford, IL; MW 131.18; 27.2 mg; 0.2 mmoles) was added to 150 parts of deionized water to form a 51.8 mM solution. 40 Parts by volume of the MnCl$_2$ solution were added to 154 parts of above formed amino acid solution to give, instantaneously, a non-chelate coordination compound solution of [manganese(II) $\cdot$ (H$_2$O)$_2$ $\cdot$ (L-isoleucine)$_2$]$^{+2}$(Cl$_2$)$^{-2}$.

The paramagnetic relaxation properties of manganese-bis-l-isoleucine in water were measured at 10 MHz (0.15T) with a spin analyzer (RADX; Houston, TX) at 37°C. After thermal equilibrium, the spin-lattice (T$_1$) and spin-spin (T$_2$) relaxation values were measured in triplicate. The T$_1$ values of a 0.99 mM solution were 109 +/- 7 msec (means +/- standard deviation) and the T$_2$ values were 28 +/- 1 msec. These values permitted the calculation of millimolar relaxivity values of 9.66 mM$^{-1}$sec$^{-1}$ for T$_1$ and 37.6 mM$^{-1}$sec$^{-1}$ for T$_2$.

The paramagnetic relaxation properties were also determined in human plasma at 37°C. The title compound was added to human plasma at a 90 μM final concentration (the total dilution of the plasma was 10.5%). The plasma remained clear yellow. The T$_1$ value (in triplicate) was 235 +/- 7 msec, the T$_2$ value was 182 +/- 5 msec, the R$_1$ was 47.3 mM$^{-1}$sec$^{-1}$, and the R$_2$ was 61.1 mM$^{-1}$sec$^{-1}$. These values were

stable based on a repeat measurement (80 minutes later) which indicated $T_1$ changed by 2% and $T_2$ changed by 8%. Thus, the $T_1$ relaxivity of [manganese(II)$\cdot$(H$_2$O)$_2$$\cdot$(L-isoleucine)$_2$]$^{+2}$(Cl$_2$)$^{-2}$ in plasma is 9.9-fold greater than gadolinium diethylenetriaminepentaacetic acid chelate (GdDTPA). The $T_2$ relaxivity is 12.7-fold greater than GdDTPA in plasma.

## EXAMPLE 6

$$[Manganese(II)\cdot(H_2O)_2\cdot(L\text{-}tyrosine)_2]^{+}{}^2(Cl_2)^{-2}$$

A non-chelate coordination compound solution of [manganese(II)$\cdot$(H$_2$O)$_2$$\cdot$(L-tyrosine)$_2$]$^{+2}$(Cl$_2$)$^{-2}$ was formed in aqueous media at room temperature. Manganese chloride (Baker analytical grade 99.5% purity, lot #434113 MnCl$_2\cdot$4H$_2$O; MW 197.07; 1.98 gm; 10 mmoles) was dissolved in 100 ml of deionized water to form a 100 mM clear solution. L-tyrosine ($\alpha$-amino-hydroxyphenyl propionic acid) (Pierce Chemical Co., Rockford, IL; MW 181.19; 11.2 mg; 0.1 mmoles) was added to 1.250 parts of deionized water to form a 4.42 mM solution. 40 Parts by volume of the MnCl$_2$ solution were added to 1810 parts of above formed amino acid solution to give, instantaneously, a non-chelate coordination compound solution of [manganese(II)$\cdot$-(H$_2$O)$_2$$\cdot$(L-tyrosine)$_2$]$^{+2}$(Cl$_2$)$^{-2}$.

The paramagnetic relaxation properties of [manganese(II)$\cdot$(H$_2$O)$_2$$\cdot$(L-tyrosine)$_2$]$^{+2}$(Cl$_2$)$^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.68 mM solution were 148 +/- 1 msec (mean +/- standard deviation) and the $T_2$ values were 38 +/- 1 msec. These values permitted the calculation of millimolar relaxivity values of 9.94 mM$^{-1}$sec$^{-1}$ for $T_1$ and 38.7 mM$^{-1}$sec$^{-1}$ for $T_2$.

The paramagnetic relaxation properties were also determined in human plasma at 37°C. The title compound was added to human plasma at a 65 $\mu$M final concentration (the total dilution of the plasma was 10.5%). The plasma remained clear yellow. The $T_1$ value (in triplicate) was 312 +/- 20 msec, the $T_2$ value was 216 +/- 2 msec, the $R_1$ was 49.3 mM$^{-1}$sec$^{-1}$, and the $R_2$ was 71.6 mM$^{-1}$sec$^{-1}$. These values were stable based on a repeat measurement (80 minutes later) which indicated $T_1$ changed by 5% and $T_2$ changed by 9%. Thus, the $T_1$ relaxivity of [manganese(II)$\cdot$(H$_2$O)$_2$$\cdot$(L-tyrosine)$_2$]$^{+2}$(Cl$_2$)$^{-2}$ in plasma is 10.3-fold greater than gadolinium diethylenetriaminepentaacetic acid chelate (GdDTPA). The $T_2$ relaxivity is 14.8-fold greater than GdDTPA in plasma.

## EXAMPLE 7

$$[Manganese(II)\cdot(H_2O)_2\cdot(L\text{-}histidine)_2]^{+}{}^2(Cl_2)^{-2}$$

A non-chelate coordination compound solution of [manganese(II)$\cdot$(H$_2$O)$_2$$\cdot$(L-histidine)$_2$]$^{+2}$(Cl$_2$)$^{-2}$ was formed in aqueous media at room temperature. Manganese chloride (Baker analytical grade 99.5% purity, lot #434113 MnCl$_2\cdot$4H$_2$O; MW 197.07; 1.98 gm; 10 mmoles) was dissolved in 100 ml of deionized water to form a 100 mM clear solution. L-histidine (Pierce Chemical Co., Rockford, IL; MW 155.16; 56.8 mg; 0.4 mmoles) was added to 70 parts of deionized water to form a 91.5 mM solution. 40 Parts by volume of the MnCl$_2$ solution were added to 87 parts of above formed amino acid solution to give, instantaneously, a non-chelate coordination compound solution of [manganese(II)$\cdot$(H$_2$O)$_2$$\cdot$(L-histidine)$_2$]$^{+2}$(Cl$_2$)$^{-2}$.

The paramagnetic relaxation properties of [manganese(II)$\cdot$(H$_2$O)$_2$$\cdot$(L-histidine)$_2$]$^{+2}$(Cl$_2$)$^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.97 mM solution were 112 +/- 4 msec (mean +/- standard deviation) and the $T_2$ values were 29 +/- 1 msec. These values permitted the calculation of millimolar relaxivity values of 9.20 mM$^{-1}$sec$^{-1}$ for $T_1$ and 35.6 mM$^{-1}$sec$^{-1}$ for $T_2$.

The paramagnetic relaxation properties were also determined in human plasma at 37°C. The title compound was added to human plasma at a 92 $\mu M$ final concentration (the total dilution of the plasma was 10.5%). The plasma remained clear yellow. The $T_1$ value (in triplicate) was 235 +/- 2 msec, the $T_2$ value was 186 +/- 3 msec, the $R_1$ was 46.3 m$M^{-1}$sec$^{-1}$, and the $R_2$ was 58.4 m$M^{-1}$sec$^{-1}$. These values were relatively stable based on a repeat measurement (80 minutes later) which indicated $T_1$ changed by 12% and $T_2$ changed by 1%. Thus, the $T_1$ relaxivity of [manganese(II)•($H_2O)_2$•($L$-histidine)$_2$]$^{+2}(Cl_2)^{-2}$ in plasma is 9.7-fold greater than gadolinium diethylenetriaminepentaacetic acid chelate (GdDTPA). The $T_2$ relaxivity is 12.2-fold greater than GdDTPA in plasma.

## EXAMPLE 8

### $[Manganese(II)$•$(H_2O)_2$•$(L$-hydroxyproline$)_2]^{+2}(Cl_2)^{-2}$

A non-chelate coordination compound solution of [manganese(II)•($H_2O)_2$•($L$-hydroxyproline)$_2$]$^{+2}(Cl_2)^{-2}$ was formed in aqueous media at room temperature. Manganese chloride (Baker analytical grade 99.5% purity, lot #434113 MnCl$_2$•4H$_2$O; MW 197.07; 1.98 gm; 10 mmoles) was dissolved in 100 ml of deionized water to form a 100 m$M$ clear solution. $L$-hydroxyproline (4-hydroxypyrrolidine-2-carboxylic acid) (Pierce Chemical Co., Rockford, IL; MW 131.13; 52.2 mg; 0.4 mmoles) was added to 40 parts of deionized water to form a 199 m$M$ solution. 40 Parts by volume of the MnCl$_2$ solution were added to 40 parts of above formed amino acid solution to give, instantaneously, a non-chelate coordination compound solution of [manganese-(II)•($H_2O)_2$•($L$-hydroxyproline)$_2$]$^{+2}(Cl_2)^{-2}$.

The paramagnetic relaxation properties of [manganese(II)•($H_2O)_2$•($L$-hydroxyproline)$_2$]$^{+2}(Cl_2)^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.98 m$M$ solution were 111 +/- 3 msec (mean +/- standard deviation) and the $T_2$ values were 26 +/- 1 msec. These values permitted the calculation of millimolar relaxivity values of 9.19 m$M^{-1}$sec$^{-1}$ for $T_1$ and 39.3 m$M^{-1}$sec$^{-1}$ for $T_2$.

The paramagnetic relaxation properties were also determined in human plasma at 37°C. The title compound was added to human plasma at a 93 $\mu M$ final concentration (the total dilution of the plasma was 10.5%). The plasma remained clear yellow. The $T_1$ value (in triplicate) was 246 +/- 13 msec, the $T_2$ value was 189 +/- 9 msec, the $R_1$ was 43.7 m$M^{-1}$sec$^{-1}$, and the $R_2$ was 56.9 m$M^{-1}$sec$^{-1}$. These values were stable based on a repeat measurement (80 minutes later) which indicated $T_1$ did not change and $T_2$ changed by 1%. Thus, the $T_1$ relaxivity of [manganese(II)•($H_2O)_2$•($L$-hydroxyproline)$_2$]$^{+2}(Cl_2)^{-2}$ in plasma is 9.1-fold greater than gadolinium diethylenetriaminepentaacetic acid chelate (GdDTPA). The $T_2$ relaxivity is 11.9-fold greater than GdDTPA in plasma.

## EXAMPLE 9

### $[Manganése(II)$•$(H_2O)_2$•$(L$-arginine$)_2]^{+2}(Cl_2)^{-2}$

A non-chelate coordination compound solution of [manganese(II)•($H_2O)_2$•($L$-arginine)$_2$]$^{+2}(Cl_2)^{-2}$ was formed in aqueous media at room temperature. Manganese chloride (Baker analytical grade 99.5% purity, lot #434113 MnCl$_2$•4H$_2$O; MW 197.07; 1.98 gm; 10 mmoles) was dissolved in 100 ml of deionized water to form a 100 m$M$ clear solution. $L$-arginine ($\alpha$-amino-$\gamma$-guanidinovaleric acid) (Pierce Chemical Co., Rockford, IL; MW 174.20; 37.2 mg; 0.2 mmoles) was added to 70 parts of deionized water to form a 88.1 m$M$ solution. 40 Parts by volume of the MnCl$_2$ solution were added to 91 parts of above formed amino acid solution to give, instantaneously, a non-chelate coordination compound solution of [manganese(II)•($H_2O)_2$•($L$-arginine)-$_2$]$^{+2}(Cl_2)^{-2}$.

The paramagnetic relaxation properties of [manganese(II)•($H_2O)_2$•($L$-arginine)$_2$]$^{+2}(Cl_2)^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.97 m$M$ solution were 348 +/- 7 msec (mean +/- standard deviation) and the $T_2$ values were 97 +/- 4 msec. These values permitted the calculation of millimolar relaxivity values of 2.96 m$M^{-1}sec^{-1}$ for $T_1$ and 10.6 m$M^{-1}sec^{-1}$ for $T_2$.

The paramagnetic relaxation properties were also determined in human plasma at 37°C. The title compound was added to human plasma at a 92 $\mu M$ final concentration (the total dilution of the plasma was 10.5%). The plasma remained clear yellow. The $T_1$ value (in triplicate) was 439 +/- 27 msec, the $T_2$ value was 287 +/- 11 msec, the $R_1$ was 24.8 m$M^{-1}sec^{-1}$, and the $R_2$ was 37.9 m$M^{-1}sec^{-1}$. These values were modestly stable based on a repeat measurement (80 minutes later) which indicated $T_1$ changed by 8% and $T_2$ changed by 22%. Thus, the $T_1$ relaxivity of [manganese(II)•$(H_2O)_2$•$(L\text{-arginine})_2]^{+2}(Cl_2)^{-2}$ in plasma is 5.2-fold greater than gadolinium diethylenetriaminepentaacetic acid chelate (GdDTPA). The $T_2$ relaxivity is 7.9-fold greater than GdDTPA in plasma.

## EXAMPLE 10

### $[Manganese(II)•(H_2O)_2•(L\text{-glutamine})_2]^{+2}(Cl_2)^{-2}$

A non-chelate coordination compound solution of [manganese(II)•$(H_2O)_2$•$(L\text{-glutamine})_2]^{+2}(Cl_2)^{-2}$ was formed in aqueous media at room temperature. Manganese chloride (Baker analytical grade 99.5% purity, lot #434113 $MnCl_2$•$4H_2O$; MW 197.07; 1.98 gm; 10 mmoles) was dissolved in 100 ml of deionized water to form a 100 m$M$ clear solution. $L$-glutamine ($\alpha$-aminoglutaramic acid) (Pierce Chemical Co., Rockford, IL; MW 146.2; 29.5 mg; 0.2 mmoles) was added to 140 parts of deionized water to form a 50.4 m$M$ solution. 40 Parts by volume of the $MnCl_2$ solution were added to 159 parts of above formed amino acid solution to give, instantaneously, a non-chelate coordination compound solution of [manganese(II)•$(H_2O)_2$•$(L\text{-glutamine})_2]^{+2}(Cl_2)^{-2}$.

The paramagnetic relaxation properties of [manganese(II)•$(H_2O)_2$•$(L\text{-glutamine})_2]^{+2}(Cl_2)^{-2}$ in water were measured at 10 MHz (0.15T) with a spin analyzer (RADX; Houston, TX) at 37°C. After thermal equilibrium, the spin-lattice ($T_1$) and spin-spin ($T_2$) relaxation values were measured in triplicate. The $T_1$ values of a 0.95 m$M$ solution were 108 +/- 3 msec (mean +/- standard deviation) and the $T_2$ values were 27 +/- 0 msec. These values permitted the calculation of millimolar relaxivity values of 9.75 m$M^{-1}sec^{-1}$ for $T_1$ and 39.0 m$M^{-1}sec^{-1}$ for $T_2$.

The paramagnetic relaxation properties were also determined in human plasma at 37°C. The title compound was added to human plasma at a 90 $\mu M$ final concentration (the total dilution of the plasma was 10.5%). The plasma remained clear yellow. The $T_1$ value (in triplicate) was 251 +/- 18 msec, the $T_2$ value was 188 +/- 8 msec, the $R_1$ was 44.3 m$M^{-1}sec^{-1}$, and the $R_2$ was 59.1 m$M^{-1}sec^{-1}$. These values were stable based on a repeat measurement (80 minutes later) which indicated $T_1$ changed by 5% and $T_2$ did not change. Thus, the $T_1$ relaxivity of [manganese(II)•$(H_2O)_2$•$(L\text{-glutamine})_2]^{+2}(Cl_2)^{-2}$ in plasma is 9.2-fold greater than gadolinium diethylenetriaminepentaacetic acid chelate (GdDTPA). The $T_2$ relaxivity is 12.3-fold greater than GdDTPA in plasma.

## EXAMPLE 11

### $Manganese(II)•(H_2O)_2•(L\text{-asparginine})_2]^{+2}(Cl_2)^{-2}$

A non-chelate coordination compound solution of Manganese(II)•$(H_2O)_2$•$(L\text{-asparginine})_2]^{+2}(Cl_2)^{-2}$ was formed in aqueous media at room temperature. Manganese chloride (Baker analytical grade 99.5% purity, lot #434113 $MnCl_2$•$4H_2O$; MW 197.07; 1.98 gm; 10 mmoles) was dissolved in 100 ml of deionized water to form a 100 m$M$ clear solution. $L$-asparagine ($\alpha$-aminosuccinamic acid) (Pierce Chemical Co., Rockford, IL; MW 150.10; 61.6 mg; 0.2 mmoles) was added to 190 parts of deionized water to form a 34.2 m$M$ solution. 40 Parts by volume of the $MnCl_2$ solution were added to 234 parts of above formed amino acid solution to give, instantaneously, a non-chelate coordination compound solution of Manganese(II)•$(H_2O)_2$•$(L\text{-aspar-}$

ginine)$_2$]$^{+2}$(Cl$_2$)$^{-2}$.

The paramagnetic relaxation properties of manganese-*bis*-1-asparagine in water were measured at 10 MHz (0.15T) with a spin analyzer (RADX; Houston, TX) at 37°C. After thermal equilibrium, the spin-lattice (T$_1$) and spin-spin (T$_2$) relaxation values were measured in triplicate. The T$_1$ values of a 0.94 m$M$ solution were 117 +/- 5 msec (mean +/- standard deviation) and the T$_2$ values were 29 +/- 1 msec. These values permitted the calculation of millimolar relaxivity values of 9.09 m$M^{-1}$sec$^{-1}$ for T$_1$ and 36.7 m$M^{-1}$sec$^{-1}$ for T$_2$.

The paramagnetic relaxation properties were also determined in human plasma at 37°C. The title compound was added to human plasma at a 90 µ$M$ final concentration (the total dilution of the plasma was 10.5%). The plasma remained clear yellow. The T$_1$ value (in triplicate) was 244 +/- 20 msec, the T$_2$ value was 190 +/- 3 msec, the R$_1$ was 45.5 m$M^{-1}$sec$^{-1}$, and the R$_2$ was 58.5 m$M^{-1}$sec$^{-1}$. These values were stable based on a repeat measurement (80 minutes later) which indicated T$_1$ changed by 7% and T$_2$ changed by 2%. Thus, the T$_1$ relaxivity of Manganese(II)•(H$_2$O)$_2$•(L-asparginine)$_2$]$^{+2}$(Cl$_2$)$^{-2}$ in plasma is 9.5-fold greater than gadolinium diethylenetriaminepentaacetic acid chelate (GdDTPA). The T$_2$ relaxivity is 12.2-fold greater than GdDTPA in plasma.

## EXAMPLE 12

### *Manganese(II)•(H$_2$O)$_2$•(L-methionine)$_2$]$^{+2}$(Cl$_2$)$^{-2}$*

A non-chelate coordination compound solution of Manganese(II)•(H$_2$O)$_2$•(L-methionine)$_2$]$^{+2}$(Cl$_2$)$^{-2}$ was formed in aqueous media at room temperature. Manganese chloride (Baker analytical grade 99.5% purity, lot #434113 MnCl$_2$•4H$_2$O; MW 197.07; 1.98 gm; 10 mmoles) was dissolved in 100 ml of deionized water to form a 100 m$M$ clear solution. L-methionine ($\alpha$-aminomethylthiobutyric acid) (Pierce Chemical Co., Rockford, IL; MW 149.21; 29.0 mg; 0.2 mmoles) was added to 70 parts of deionized water to form a 97.2 m$M$ solution. 40 Parts by volume of the MnCl$_2$ solution were added to 82.3 parts of above formed amino acid solution to give, instantaneously, a non-chelate coordination compound solution of Manganese(II)•(H$_2$O)$_2$•(L-methionine)$_2$]$^{+2}$(Cl$_2$)$^{-2}$.

The paramagnetic relaxation properties of Manganese(II)•(H$_2$O)$_2$•(L-methionine)$_2$]$^{+2}$(Cl$_2$)$^{-2}$ in water were measured at 10 MHz (0.15T) with a spin analyzer (RADX; Houston, TX) at 37°C. After thermal equilibrium, the spin-lattice (T$_1$) and spin-spin (T$_2$) relaxation values were measured in triplicate. The T$_1$ values of a 0.97 m$M$ solution were 106 +/- 2 msec (mean +/- standard deviation) and the T$_2$ values were 27 +/- 1 msec. These values permitted the calculation of millimolar relaxivity values of 9.73 m$M^{-1}$sec$^{-1}$ for T$_1$ and 38.2 m$M^{-1}$sec$^{-1}$ for T$_2$.

The paramagnetic relaxation properties were also determined in human plasma at 37°C. The title compound was added to human plasma at a 92 µ$M$ final concentration (the total dilution of the plasma was 10.5%). The plasma remained clear yellow. The T$_1$ value (in triplicate) was 257 +/- 18 msec, the T$_2$ value was 202 +/- 9 msec, the R$_1$ was 42.3 m$M^{-1}$sec$^{-1}$, and the R$_2$ was 52.8 m$M^{-1}$sec$^{-1}$. These values were stable based on a repeat measurement (80 minutes later) which indicated T$_1$ changed by 1% and T$_2$ changed by 5%. Thus, the T$_1$ relaxivity of Manganese(II)•(H$_2$O)$_2$•(L-methionine)$_2$]$^{+2}$(Cl$_2$)$^{-2}$ in plasma is 8.8-fold greater than gadolinium diethylenetriaminepentaacetic acid chelate (GdDTPA). The T$_2$ relaxivity is 11.2-fold greater than GdDTPA in plasma.

## EXAMPLE 13

### *Manganese(II)•(H$_2$O)$_2$•(L-proline)$_2$]$^{+2}$(Cl$_2$)$^{-2}$*

A non-chelate coordination compound solution of Manganese(II)•(H$_2$O)$_2$•(L-proline)$_2$]$^{+2}$(Cl$_2$)$^{-2}$ was formed in aqueous media at room temperature. Manganese chloride (Baker analytical grade 99.5% purity, lot #434113 MnCl$_2$•4H$_2$O; MW 197.07; 1.98 gm; 10 mmoles) was dissolved in 100 ml of deionized water to form a 100 m$M$ clear solution. $L$-proline (pyrrolidine-2-carboxylic acid) (Pierce Chemical Co., Rockford, IL; MW 115.13; 29.2 mg; 0.3 mmoles) was added to 70 parts of deionized water to form a 126.8 m$M$ solution. 40 Parts by volume of the MnCl$_2$ solution were added to 63 parts of above formed amino acid solution to give, instantaneously, a non-chelate coordination compound solution of Manganese(II)$\cdot$(H$_2$O)$_2$$\cdot$($L$-proline)$_2$]$^{+2}$(Cl$_2$)$^{-2}$.

The paramagnetic relaxation properties of Manganese(II)$\cdot$(H$_2$O)$_2$$\cdot$($L$-proline)$_2$]$^{+2}$(Cl$_2$)$^{-2}$ in water were measured by 10 MHz (0.15T) with a spin analyzer (RADX; Houston, TX) at 37°C. After thermal equilibrium, the spin-lattice (T$_1$) and spin-spin (T$_2$) relaxation values were measured in triplicate. The T$_1$ values of a 0.98 m$M$ solution were 106 +/-4 msec (mean +/- standard deviation) and the T$_2$ values were 26 +/- 2 msec. These values permitted the calculation of millimolar relaxivity values of 9.63 m$M^{-1}$sec$^{-1}$ for T$_1$ and 39.3 m$M^{-1}$sec$^{-1}$ for T$_2$.

The paramagnetic relaxation properties were also determined in human plasma at 37°C. The title compound was added to human plasma at a 93 µ$M$ final concentration (the total dilution of the plasma was 10.5%). The plasma remained clear yellow. The T$_1$ value (in triplicate) was 234 +/- 12 msec, the T$_2$ value was 202 +/- 12 msec, the R$_1$ was 46.5 m$M^{-1}$sec$^{-1}$, and the R$_2$ was 53.2 m$M^{-1}$sec$^{-1}$. These values were stable based on a repeat measurement (80 minutes later) which indicated T$_1$ changed by 5% and T$_2$ changed by 12%. Thus, the T$_1$ relaxivity of Manganese(II)$\cdot$(H$_2$O)$_2$$\cdot$($L$-proline)$_2$]$^{+2}$(Cl$_2$)$^{-2}$ in plasma is 9.7-fold greater than gadolinium diethylenetriaminepentaacetic acid chelate (GdDTPA). The T$_2$ relaxivity is 11.1-fold greater than GdDTPA in plasma.

## EXAMPLE 14

### Manganese(II)$\cdot$(H$_2$O)$_2$$\cdot$(L-serine)$_2$]$^{+2}$(Cl$_2$)$^{-2}$

A non-chelate coordination compound solution of Manganese(II)$\cdot$(H$_2$O)$_2$$\cdot$($L$-serine)$_2$]$^{+2}$(Cl$_2$)$^{-2}$ was formed in aqueous media at room temperature. Manganese chloride (Baker analytical grade 99.5% purity, lot #434113 MnCl$_2$$\cdot$4H$_2$O; MW 197.07; 1.98 gm; 10 mmoles) was dissolved in 100 ml of deionized water to form a 100 m$M$ clear solution. $L$-serine ($\alpha$-amino-$\beta$-hydroxypropionic acid) (Pierce Chemical Co., Rockford, IL; MW 105.09; 53.3 mg; 0.5 mmoles) was added to 40 parts of deionized water to form a 253.6 m$M$ solution. 40 Parts by volume of the MnCl$_2$ solution were added to 31.5 parts of above formed amino acid solution to give, instantaneously, a non-chelate coordination compound solution of Manganese(II)$\cdot$(H$_2$O)$_2$$\cdot$($L$-serine)$_2$]$^{+2}$(Cl$_2$)$^{-2}$.

The paramagnetic relaxation properties of Manganese(II)$\cdot$(H$_2$O)$_2$$\cdot$($L$-serine)$_2$]$^{+2}$(Cl$_2$)$^{-2}$ in water were measured at 10 MHz (0.15T) with a spin analyzer (RADX; Houston, TX) at 37°C. After thermal equilibrium, the spin-lattice (T$_1$) and spin-spin (T$_2$) relaxation values were measured in triplicate. The T$_1$ values of a 0.989 m$M$ solution were 111 +/- 2 msec (mean +/- standard deviation) and the T$_2$ values were 27 +/- 1 msec. These values permitted the calculation of millimolar relaxivity values of 9.19 m$M^{-1}$sec$^{-1}$ for T$_1$ and 37.8 m$M^{-1}$sec$^{-1}$ for T$_2$.

The paramagnetic relaxation properties were also determined in human plasma at 37°C. The title compound was added to human plasma at a 93 µ$M$ final concentration (the total dilution of the plasma was 10.5%). The plasma remained clear yellow. The T$_1$ value (in triplicate) was 238 +/- 9 msec, the T$_2$ value was 189 +/- 6 msec, the R$_1$ was 45.2 m$M^{-1}$sec$^{-1}$, and the R$_2$ was 56.9 m$M^{-1}$sec$^{-1}$. These values were stable based on a repeat measurement (80 minutes later) which indicated T$_1$ changed by 3% and T$_2$ changed by 2%. Thus, the T$_1$ relaxivity of Manganese(II)$\cdot$(H$_2$O)$_2$$\cdot$($L$-serine)$_2$]$^{+2}$(Cl$_2$)$^{-2}$ in plasma is 9.4-fold greater than gadolinium diethylenetriaminepentaacetic acid chelate (GdDTPA). The T$_2$ relaxivity is 11.9-fold greater than GdDTPA in plasma.

## EXAMPLE 15

*Manganese(II)• (H₂O)₂ • (L-threonine)₂]⁺²(Cl₂)⁻²*

A non-chelate coordination compound solution of Manganese(II)$\cdot$(H$_2$O)$_2$ $\cdot$($L$-threonine)$_2$]$^{+}$2(Cl$_2$)$^{-2}$ was formed in aqueous media at room temperature. Manganese chloride (Baker analytical grade 99.5% purity, lot #434113 MnCl$_2 \cdot$4H$_2$O; MW 197.07; 1.98 gm; 10 mmoles) was dissolved in 100 ml of deionized water to form a 100 m$M$ clear solution. $L$-threonine ($\alpha$-amino-$\beta$-hydroxybutyric acid) (Pierce Chemical Co., Rockford, IL; MW 119.12; 25.6 mg; 0.2 mmoles) was added to 80 parts of deionized water to form a 107.5 m$M$ solution. 40 Parts by volume of the MnCl$_2$ solution were added to 74.4 parts of above formed amino acid solution to give, instantaneously, a non-chelate coordination compound solution of Manganese(II)$\cdot$(H$_2$O)$_2$ $\cdot$-($L$-threonine)$_2$]$^{+}$2(Cl$_2$)$^{-2}$.

The paramagnetic relaxation properties of Manganese(II)$\cdot$(H$_2$O)$_2$ $\cdot$($L$-threonine)$_2$]$^{+}$2(Cl$_2$)$^{-2}$ in water were measured at 10 MHz (0.15T) with a spin analyzer (RADX; Houston, TX) at 37$^\circ$ C. After thermal equilibrium, the spin-lattice (T$_1$) and spin-spin (T$_2$) relaxation values were measured in triplicate. The T$_1$ values of a 0.97 m$M$ solution were 105 +/- 1 msec (mean +/- standard deviation) and the T$_2$ values were 28 +/- 1 msec. These values permitted the calculation of millimolar relaxivity values of 9.82 m$M^{-1}$sec$^{-1}$ for T$_1$ and 36.8 m$M^{-1}$sec$^{-1}$ for T$_2$.

The paramagnetic relaxation properties were also determined in human plasma at 37$^\circ$ C. The title compound was added to human plasma at a 92 $\mu M$ final concentration (the total dilution of the plasma was 10.5%). The plasma remained clear yellow. The T$_1$ value (in triplicate) was 241 +/- 7 msec, the T$_2$ value was 196 +/- 17 msec, the R$_1$ was 45.1 m$M^{-1}$sec$^{-1}$, and the R$_2$ was 55.5 m$M^{-1}$sec$^{-1}$. These values were stable based on a repeat measurement (80 minutes later) which indicated T$_1$ changed by 1% and T$_2$ changed by 11%. Thus, the T$_1$ relaxivity of Manganese(II)$\cdot$(H$_2$O)$_2$ $\cdot$($L$-threonine)$_2$]$^{+}$2(Cl$_2$)$^{-2}$ in plasma is 9.4-fold greater than gadolinium diethylenetriaminepentaacetic acid chelate (GdDTPA). The T$_2$ relaxivity if 11.6-fold greater than GdDTPA in plasma.

## EXAMPLE 16

*Manganese(II)• (H₂O)₂ • (L-lysine)₂]⁺²(Cl₂)⁻²*

A non-chelate coordination compound solution of Manganese(II)$\cdot$(H$_2$O)$_2$ $\cdot$($L$-lysine)$_2$]$^{+}$2(Cl$_2$)$^{-2}$ was formed in aqueous media at room temperature. Manganese chloride (Baker analytical grade 99.5% purity, lot #434113 MnCl$_2 \cdot$4H$_2$O; MW 197.07; 1.98 gm; 10 mmoles) was dissolved in 100 ml of deionized water to form a 100 m$M$ clear solution. $L$-lysine ($\alpha$,$\gamma$-diaminocaproic acid) (Pierce Chemical Co., Rockford, IL; MW 182.70; 42.4 mg; 10 mmoles) was added to 50 parts of deionized water to form a 166.0 m$M$ solution. 40 Parts by volume of the MnCl$_2$ solution were added to 69 parts of above formed amino acid solution to give, instantaneously, a non-chelate coordination compound solution of Manganese(II)$\cdot$(H$_2$O)$_2$ $\cdot$($L$-lysine)$_2$]$^{+}$2(Cl$_2$)-$^{-2}$.

The paramagnetic relaxation properties of Manganese(II)$\cdot$(H$_2$O)$_2$ $\cdot$($L$-lysine)$_2$]$^{+}$2(Cl$_2$)$^{-2}$ in water were measured at 10 MHz (0.15T) with a spin analyzer (RADX; Houston, TX) at 37$^\circ$ C. After thermal equilibrium, the spin-lattice (T$_1$) and spin-spin (T$_2$) relaxation values were measured in triplicate. The T$_1$ values of a 0.97 m$M$ solution were 108 +/-9 msec (mean +/- standard deviation) and the T$_2$ values were 26 +/- 1 msec. These values permitted the calculation of millimolar relaxivity values of 9.55 m$M^{-1}$sec$^{-1}$ for T$_1$ and 39.7 m$M^{-1}$sec$^{-1}$ for T$_2$.

The paramagnetic relaxation properties were also determined in human plasma at 37$^\circ$ C. The title compound was added to human plasma at a 92 $\mu M$ final concentration (the total dilution of the plasma was 10.5%). The plasma remained clear yellow. The T$_1$ value (in triplicate) was 230 +/- 9 msec, the T$_2$ value was 192 +/- 8 msec, the R$_1$ was 47.3 m$M^{-1}$sec$^{-1}$, and the R$_2$ was 56.6 m$M^{-1}$sec$^{-1}$. These values were stable based on a repeat measurement (80 minutes later) which indicated T$_1$ changed by 3% and T$_2$ changed by 3%. Thus, the T$_1$ relaxivity of Manganese(II)$\cdot$(H$_2$O)$_2$ $\cdot$($L$-lysine)$_2$]$^{+}$2(Cl$_2$)$^{-2}$ in plasma is 9.9-fold greater than gadolinium diethylenetriaminepentaacetic acid chelate (GdDTPA). The T$_2$ relaxivity is 11.8-fold greater than GdDTPA in plasma.

## EXAMPLE 17

*Manganese(II)• (H₂O)₂ • (L-aspartic acid)₂]⁺²(Cl₂)⁻²*

A non-chelate coordination compound solution of Manganese(II)•(H₂O)₂•(*L*-aspartic acid)₂]⁺²(Cl₂)⁻² was formed in aqueous media at room temperature. Manganese chloride (Baker analytical grade 99.5% purity, lot #434113 MnCl₂•4H₂O; MW 197.07; 1.98 gm; 10 mmoles) was dissolved in 100 ml of deionized water to form a 100 m*M* clear solution. *L*-aspartic acid (Pierce Chemical Co., Rockford, IL; MW 133.10; 23.0 mg; 0.2 mmoles) was added to 390 parts of deionized water to form a 19.2 m*M* solution. 40 Parts by volume of the MnCl₂ solution were added to 417 parts of above formed amino acid solution to give, instantaneously, a non-chelate coordination compound solution of Manganese(II)•(H₂O)₂•(*L*-aspartic acid)₂]⁺²(Cl₂)⁻².

The paramagnetic relaxation properties of Manganese(II)•(H₂O)₂•(*L*-aspartic acid)₂]⁺²(Cl₂)⁻² in water were measured at 10 MHz (0.15T) with a spin analyzer (RADX; Houston, TX) at 37°C. After thermal equilibrium, the spin-lattice (T₁) and spin-spin (T₂) relaxation values were measured in triplicate. The T₁ values of a 0.90 m*M* solution were 116 +/- 3 msec (mean +/- standard deviation) and the T₂ values were 28 +/- 1 msec. These values permitted the calculation of millimolar relaxivity values of 9.58 m*M*⁻¹sec⁻¹ for T₁ and 39.7 m*M*⁻¹sec⁻¹ for T₂.

The paramagnetic relaxation properties were also determined in human plasma at 37°C. The title compound was added to human plasma at a 86 μ*M* final concentration (the total dilution of the plasma was 10.5%). The plasma remained clear yellow. The T₁ value (in triplicate) was 251 +/- 12 msec, the T₂ value was 190 +/- 12 msec, the R₁ was 46.3 m*M*⁻¹sec⁻¹, and the R₂ was 61.2 m*M*⁻¹sec⁻¹. These values were stable based on a repeat measurement (80 minutes later) which indicated T₁ did not change and T₂ changed by 3%. Thus, the T₁ relaxivity of Manganese(II)•(H₂O)₂•(*L*-aspartic acid)₂]⁺²(Cl₂)⁻² in plasma is 9.7-fold greater than gadolinium diethylenetriaminepentaacetic acid chelate (GdDTPA). The T₂ relaxivity if 12.8-fold greater than GdDTPA in plasma.

## EXAMPLE 18

*Manganese(II)• (H₂O)₂ • (L-valine)₂]⁺²(Cl₂)⁻²*

A non-chelate coordination compound solution of Manganese(II)•(H₂O)₂•(*L*-valine)₂]⁺²(Cl₂)⁻² was formed in aqueous media at room temperature. Manganese chloride (Baker analytical grade 99.5% purity, lot #434113 MnCl₂•4H₂O; MW 197.07; 1.98 gm; 10 mmoles) was dissolved in 100 ml of deionized water to form a 100 m*M* clear solution. *L*-valine (α-aminoisovaleric acid) (Pierce Chemical Co., Rockford, IL; MW 117.15; 26.2 mg; 0.2 mmoles) was added to 80 parts of deionized water to form a 111.8 m*M* solution. 40 Parts by volume of the MnCl₂ solution were added to 72 parts of above formed amino acid solution to give, instantaneously, a non-chelate coordination compound solution of Manganese(II)•(H₂O)₂•(*L*-valine)₂]⁺²(Cl₂)⁻².

The paramagnetic relaxation properties of Manganese(II)•(H₂O)₂•(*L*-valine)₂]⁺²(Cl₂)⁻² in water were measured at 10 MHz (0.15T) with a spin analyzer (RADX; Houston, TX) at 37°C. After thermal equilibrium, the spin-lattice (T₁) and spin-spin (T₂) relaxation values were measured in triplicate. The T₁ values of a 0.97 m*M* solution were 113 +/-10 msec (mean +/- standard deviation) and the T₂ values were 26 +/- 1 msec. These values permitted the calculation of millimolar relaxivity values of 9.12 m*M*⁻¹sec⁻¹ for T₁ and 39.7 m*M*⁻¹sec⁻¹ for T₂.

The paramagnetic relaxation properties were also determined in human plasma at 37°C. The title compound was added to human plasma at a 92 μ*M* final concentration (the total dilution of the plasma was 10.5%). The plasma remained clear yellow. The T₁ value (in triplicate) was 250 +/- 2 msec, the T₂ value was 191 +/- 8 msec, the R₁ was 43.5 m*M*⁻¹sec⁻¹, and the R₂ was 56.9 m*M*⁻¹sec⁻¹. These values were stable based on a repeat measurement (80 minutes later) which indicated $T_1$ changed by 4% and $T_2$ changed by 4%. Thus, the $T_1$ relaxivity of Manganese(II)$\cdot$(H$_2$O)$_2\cdot$(L-valine)$_2$]$^{+2}$(Cl$_2$)$^{-2}$ in plasma is 9.1-fold greater than gadolinium diethylenetriaminepentaacetic acid chelate (GdDTPA). The $T_2$ relaxivity is 11.9-fold greater than GdDTPA in plasma.

## EXAMPLE 19

### Manganese(II)$\cdot$(H$_2$O)$_2\cdot$(L-sarcosine)$_2$]$^{+2}$(Cl$_2$)$^{-2}$

A non-chelate coordination compound solution of Manganese(II)$\cdot$(H$_2$O)$_2\cdot$(L-sarcosine)$_2$]$^{+2}$(Cl$_2$)$^{-2}$ was formed in aqueous media at room temperature. Manganese chloride (Baker analytical grade 99.5% purity, lot #434113 MnCl$_2\cdot$4H$_2$O; MW 197.07; 1.98 gm; 10 mmoles) was dissolved in 100 ml of deionized water to form a 100 m$M$ clear solution. L-sarcosine (N-methylglycine) (Pierce Chemical Co., Rockford, IL; MW 89.09; 62.9 mg; 0.7 mmoles) was added to 30 parts of deionized water to form a 353 m$M$ solution. 40 Parts by volume of the MnCl$_2$ solution were added to 23 parts of above formed amino acid solution to give, instantaneously, a non-chelate coordination compound solution of Manganese(II)$\cdot$(H$_2$O)$_2\cdot$(L-sarcosine)$_2$]$^{+2}$-(Cl$_2$)$^{-2}$.

The paramagnetic relaxation properties of Manganese(II)$\cdot$(H$_2$O)$_2\cdot$(L-sarcosine)$_2$]$^{+2}$(Cl$_2$)$^{-2}$ in water were measured at 10 MHz (0.15T) with a spin analyzer (RADX; Houston, TX) at 37° C. After thermal equilibrium, the spin-lattice ($T_1$) and spin-spin ($T_2$) relaxation values were measured in triplicate. The $T_1$ values of a 0.98 m$M$ solution were 111 +/- 2 msec (mean +/- standard deviation) and the $T_2$ values were 26 +/- 1 msec. These values permitted the calculation of millimolar relaxivity values of 9.19 m$M^{-1}$sec$^{-1}$ for $T_1$ and 39.3 m$M^{-1}$sec$^{-1}$ for $T_2$.

The paramagnetic relaxation properties were also determined in human plasma at 37° C. The title compound was added to human plasma at a 93 $\mu M$ final concentration (the total dilution of the plasma was 10.5%). The plasma remained clear yellow. The $T_1$ value (in triplicate) was 232 +/- 2 msec, the $T_2$ value was 194 +/- 16 msec, the $R_1$ was 46.4 m$M^{-1}$sec$^{-1}$, and the $R_2$ was 55.4 m$M^{-1}$sec$^{-1}$. These values were stable based on a repeat measurement (80 minutes later) which indicated $T_1$ changed by 6% and $T_2$ changed by 4%. Thus, the $T_1$ relaxivity of Manganese(II)$\cdot$(H$_2$O)$_2\cdot$(L-sarcosine)$_2$]$^{+2}$(Cl$_2$)$^{-2}$ in plasma is 9.7-fold greater than gadolinium diethylenetriaminepentaacetic acid chelate (GdDTPA). The $T_2$ relaxivity is 11.5-fold greater than GdDTPA in plasma.

## EXAMPLE 20

### Manganese(II)$\cdot$(H$_2$O)$_2\cdot$(L-cysteine)$_2$]$^{+2}$(Cl$_2$)$^{-2}$

A non-chelate coordination compound solution of Manganese(II)$\cdot$(H$_2$O)$_2\cdot$(L-cysteine)$_2$]$^{+2}$(Cl$_2$)$^{-2}$ was formed in aqueous media at room temperature. Manganese chloride (Baker analytical grade 99.5% purity, lot #434113 MnCl$_2\cdot$4H$_2$O; MW 197.07; 1.98 gm; 10 mmoles) was dissolved in 100 ml of deionized water to form a 100 m$M$ clear solution. L-cysteine ($\alpha$-amino-$\beta$-thiolpropionic acid) (Pierce Chemical Co., Rockford, IL; MW 175.62; 41.4 mg; 0.2 mmoles) was added to 50 parts of deionized water to form a 117.9 m$M$ solution. 40 Parts by volume of the MnCl$_2$ solution were added to 67.8 parts of above formed amino acid solution to give, instantaneously, a non-chelate coordination compound solution of Manganese(II)$\cdot$(H$_2$O)$_2\cdot$(L-cysteine)-$_2$]$^{+2}$(Cl$_2$)$^{-2}$.

The paramagnetic relaxation properties of Manganese(II)$\cdot$(H$_2$O)$_2\cdot$(L-cysteine)$_2$]$^{+2}$(Cl$_2$)$^{-2}$ in water were measured at 10 MHz (0.15T) with a spin analyzer (RADX; Houston, TX) at 37° C. After thermal equilibrium, the spin-lattice ($T_1$) and spin-spin ($T_2$) relaxation values were measured in triplicate. The $T_1$ values of a 0.97 m$M$ solution were 105 +/-4 msec (mean +/- standard deviation) and the $T_2$ values were 25 +/- 1 msec. These values permitted the calculation of millimolar relaxivity values of 9.82 m$M^{-1}$sec$^{-1}$ for $T_1$ and 41.2 m$M^{-1}$sec$^{-1}$ for $T_2$.

The paramagnetic relaxation properties were also determined in human plasma at 37° C. The title compound was added to human plasma at a 92 $\mu M$ final concentration (the total dilution of the plasma was 10.5%). The plasma remained clear yellow. The $T_1$ value (in triplicate) was 247 +/- 14 msec, the $T_2$ value was 190 +/- 17 msec, the $R_1$ was 44.0 m$M^{-1}$sec$^{-1}$, and the $R_2$ was 57.2 m$M^{-1}$sec$^{-1}$. These values were stable based on a repeat measurement (80 minutes later) which indicated $T_1$ changed by 4% and $T_2$ changed by 1%. Thus, the $T_1$ relaxivity of Manganese(II)$^{\bullet}$(H$_2$O)$_2$$^{\bullet}$($L$-cysteine)$_2$]$^{+2}$(Cl$_2$)$^{-2}$ in plasma is 9.2-fold greater than gadolinium diethylenetriaminepentaacetic acid chelate (GdDTPA). The $T_2$ relaxivity is 11.9-fold greater than GdDTPA in plasma.

## EXAMPLE 21

### *Other Mn(II) Coordination Compounds*

Repeating the procedure of Example 1 but replacing $L$-alanine with an equamolar amount of the following naturally occurring amino acids described in HANDBOOK OF BIOCHEMISTRY, (supra, pp B4-B51) and the literature references cited therein yields a solution of the corresponding [manganese(II)$^{\bullet}$(H$_2$O)$_2$$^{\bullet}$(amino acid)$_2$](dianion) coordination compound which yields greatly enhanced relaxivity in NMR imaging: $\beta$-alanine, $\alpha$-aminobutyric acid, $\delta$-aminobutyric acid, 1-aminocyclopropane-1-carboxylic acid, 2-amino-3-formyl-3-pentenoic acid, $\alpha$-aminoheptanoic acid, 2-amino-4-hexenoic acid, $\alpha$-aminoisobutyric acid, $\beta$-aminoisobutyric acid, $\gamma$-amino-$\alpha$-methylenebutyric acid, 2-amino-4-methylhexanoic acid, 2-amino-4-methylhex-4-enoic acid, 2-amino-5-methylhex-4-enoic acid , $\alpha$-aminooctanoic acid, hypoglycin A, $\alpha$-(methylenecyclopropyl)-glycine, $\beta$-(methylenecyclopropyl)-$\beta$-methylalanine, $\alpha$-aminoadipic acid, 3-aminoglutaric acid, $\alpha$-aminopimelic acid, ethylasparagine, $\gamma$-ethylideneglutamic acid, $N$-fumarylalanine, N$^5$-isopropylglutamine, N$^4$-methylasparagine, $\beta$-methylaspartic acid, $\gamma$-methylglutamic acid, $\gamma$methyleneglutamic acid, theanine, $N$-acetylornithine, $\alpha$-amino-$\gamma$-$N$-acetylaminobutyric acid, N-$\epsilon$-(2-amino-2-carboxyethyl)-lysine, $N$-$\delta$-(2-amino-2-carboxyethyl)-ornithine, 2-amino-3-dimethylaminopropionic acid, $\alpha$-amino-$\beta$-methylaminopropionic acid, canaline, $\alpha,\delta$-diaminobutyric acid, 3,5-diaminohexanoic acid, $\alpha,\delta$-diaminopropionic acid, lathyrus factor, $\beta$-lysine, lysopine, $\epsilon$-$N$-methyllysine, ornithine, $\beta$-$N$-Oxalyl-$\alpha,\beta$-diaminopropionic acid, acetylenic dicarboxylic acid diamine, $\alpha,\epsilon$-diaminopimelic acid, 2,3-diaminosuccinic acid, $O$-acetylhomoserine, 2-amino-4,5-dihydroxypentanoic acid, $\alpha$-amino-$\gamma$-hydroxyadipic acid, 2-amino-6-hydroxyaminohexanoic acid, $\alpha$-amino-$\gamma$hydroxybutric acid, 2-amino-6-hydroxy-4-methylhex-4-enoic acid, 2-amino-3-hydroxymethyl-3-pentenoic acid, $\alpha$-amino-$\gamma$-hydroxypimelic acid, $\alpha$-amino-$\delta$-hydroxyvaleric acid, $\alpha$-amino-$\beta$-ketobutyric acid, $\alpha$-amino-$\beta$-methyl-$\gamma$-,$\delta$-dihydroxyisocaproic acid, $O$-butylhomoserine, $\beta,\gamma$-dihydroxyglutamic acid, $\beta,\gamma$-dihydroxyisoleucine, $\gamma,\delta$-dihydroxyleucine, $O$-ethylhomoserine, homoserine, $\alpha$-hydroxyalanine, $\alpha$-hydroxy-$\gamma$-aminobutyric acid, $\beta$-hydroxy-$\gamma$-aminobutyric acid, $\alpha$-hydroxy-$\epsilon$-aminocaproic acid, $\beta$-hydroxyasparagine, $\beta$-hydroxyaspartic acid, N$^4$-(2-hydroxyethyl)-asparagine, N$^5$-(2-hydroxyethyl)-glutamine, $\beta$-hydroxyglutamic acid, $\gamma$-hydroxyglutamic acid, $\gamma$-hydroxyglutamine, $\epsilon$-hydroxyaminonorleucine, $\delta$-hydroxy-$\gamma$-ketonorvaline, $\delta$-hydroxyleucenine, $\beta$-hydroxyleucine, $\delta$-hydroxyleucine, threo-$\beta$-hydroxyleucine, $\alpha$-hydroxylysine, $\delta$-hydroxylysine, $\gamma$-hydroxynorvaline, $\alpha$-hydroxyvaline, $\gamma$-hydroxyvaline, 4-ketonorleucine, $\gamma$-methyl-$\gamma$-hydroxyglutamic acid, pantonine, $O$-propylhomoserine, $O$-succinylhomoserine, tabtoxinine, $\alpha$-amino-$\beta$-phenylbutyric acid, 3-carboxy-4-hydroxyphenylalanine, $m$carboxylphenylalanine, 2,4-dihydroxyphenylalaine, 3,5-dihydroxyphenylglycine, 3-hydroxykynurenine, $m$-hydroxyphenylglycine, kynurenine, $O$-methyltyrosine, $m$-tyrosine, albizzline, canavanine, canavanosuccinic acid, citrulline, desaminocanavanine, gigartinine, homoarginine, homocitrulline, $\gamma$-hydroxyarginine, $\gamma$-hydroxyhomoarginine, indospicine, alanosine, azaserine, $\beta$-cyanoalanine, ee-diazo, hadacidin, *allo*-hydroxyproline, *allo*-kainic acid, 4-aminopipecolic acid, ascorbigen, azetidine-2-carboxylic acid, baikiain, cucurbitine, dihydrozanthurenic acid, demoic acid, echinine, guvacine, 4-hydroxy-4-methylproline, hydroxyminaline, 4-hydroxypipecolic acid, 5-hydroxypipecolic acid, 3-hydroxyproline, 4-hydroxyproline, 2-hydrotryptophan, 5-hydroxytryptophan, ibotenic acid, 40imidazoleacetic acid, $N$-(indole-3-acetyl)-aspartic acid, indol-3-acetyl-ee-lysine, kainic acid, 4-keto-5-methylproline, 4-ketopipecolic acid, 4-ketoproline, lathytine, 4-methyleneproline, 4-methylproline, $N$-methyl-proline, $\beta$-methyltryptophan, mimosine, minaline, muscazone, $\beta$-3-oxidolylalanine, pipecolic acid, $\beta$-pyrazolylalanine, roseanine, stizolobic acid, stizologinic acid, tricholomic acid, viomycidine, willardine, abine, 2,3-dihydro-3,3-dimethyl-1$H$-pyrrolo-[1,2-$\alpha$]indole-9-alanine, $\beta$-$N$-dimethylleucine, $\gamma$-formyl-$N$-methylnorvaline, fusarinine, homarine, 4-hydroxy-$N$-methylproline, merodesmosine, $N$methylalanine, 1-methylhistidine, 3-methylhistidine, N-methylisoleucine, N-methylleucine, N-methyl-β-methylleucine, N-methyl-O-methylserine, Nmethylphenylglycine, N-methyltyrosine, l-methylvaline, sacchropine, N-acetyldjenkolic acid, alliin, S-allylcystein, S-allylmercaptocystein, α-amino-β-(2-amino-2-carboxyethylmercaptobutyric acid), cystathionine, 3-amino-(3-carboxypropyldimethylsulfonium), carbamyltaurine, 2-[S-(β-carboxy-β-aminoethyltryptophan)],S-(β-carboxylethyl)-cysteine, N,(1-carboxylethyl)-taurine, S-(2-carboxyisopropyl)-cystein, S-)2-carboxylpropyl)-cystein, chondrine, cycloallin, cysteic acid, cystein sulfinic acid, cystine, disulfoxide, S-(1,2-dicarboxyethyl)-cystein, dichrostachinic acid, dihydroallin, djenkolic acid, ethionine, felinine, N-formylmethionine, glucobrassicin, guanidotaurine, homocystein, homocystine, homocysteinecystein disulfide, homolanthionine, homomethionine, hypotaurine, isovalthine, lanthionine, S-methylcystein, 3,3'-(2-methylethylene-1,2-dithio)-dialanine, β-methyllanthione, S-methylmethionine, β-methylselenoalanine, neoglucobrassicin, S-(prop-1-enyl)-cystein, S-(prop-1-enyl)-cysteine sulfoxide, S-n-propylcystein, S-n-propylcysteine sulfoxide, selenocystathionine, selenocystine, selenomethylselenocystein, taurine, β-(2-thioazole)-β-alanine, thiohistidine, thiostreptine, tyrosine-O-sulfate, 2-amino-4,4-dichlorobutyric acid, 3,5-dibromotyrosine, 3,3'-diiodothyronine, 3,5-diiodotyrosine, 3-monobromotyrosine, 2-monoiodohistidine, monoiodotyrosine, thyroxine, 3,5,3'-triiodothyronine, α-amino-β-phosphonopropionic acid, ciliatine, 2-dimethylaminoethylphosphonic acid, lombricine, 2-methylaminoethylphosphonic acid, O-phosphohomoserine, O-phosphoserine, 2-trimethylaminoethylbetainephosphonic acid, N-(3-amino-3-carboxypropyl)-β-carboxypyridinium betaine, betonicine, γ-butyrobetaine, carnitine, desmosine, ergothionine, hercyin, homobetainehomostachydrine, 3-hydroxystachydrine, hypaphorin, isodesmosine, laminine, lycin, miokinine, nicotianine, stachydrine, trigonelline, ee-N-trimethyllysine betaine, D-allothreonine, 1-amino-D-proline, D-(3-carboxy-4-hydroxyphenyl)-glycine, D-cycloserine, m-carboxyphenylglycine, N-α-malonyl-D-methionine, D-α-methylserine, D-octopine, D-penicillamine, and turcine.

## EXAMPLE 22

### Toxicity of [Manganese(II) · (H₂O)₂ · (glycine)₂]⁺²(Cl₂)⁻²

Rats were injected with various doses of [manganese(II)·(H₂O)₂·(glycine)₂]⁺²(Cl₂)⁻² prepared in accordance with the procedure of Example 3 to obtain an $LD_{50}$ (dosage required to kill 50% of the population of animals) under standard conditions. The doses were 0.3, 0.5, and 0.8 mmole/kg. 10 Animals were used in each dosage group. The $LD_{50}$ was found to be 0.70 mmole/kg, and the 95% confidence limits were 0.57 to 0.93 mmole/kg. The $LD_{50}$ of manganese chloride IV under the same conditions is less than 0.2 mmole/kg.

## EXAMPLE 23

### Stability of [Manganese(II) · (H₂O)₂ · (glycine)₂]⁺²(Cl₂)⁻²

NMR contrast agents must be stable in solution under adverse conditions to be effective as pharmaceutical agents. Aqueous solutions of [manganese(II)·(H₂O)₂·(glycine)₂]⁺²(Cl₂)⁻² and [manganese(II)·(H₂O)₂·(gluconate)₂]⁺²(Cl₂)⁻² were tested for stability after addition of a NaOH solution.

[Manganese(II)·(H₂O)₂·(glycine)₂]⁺²(Cl₂)⁻² was prepared in accordance with the procedure of Example 3. One ml of a 161 mM solution (initial pH 5) was placed in a test tube at 19° C. Sodium hydroxide (0.032 ml; 1 N) was added to the solution, giving a ratio of OH⁻ to compound of 0.2. Although the pH increased to above 7.0, the solution remained clear with no discoloration or formation of a precipitate.

[Manganese(II)·(H₂O)₂·(gluconate)₂]⁺²(Cl₂)⁻² was prepared according to the procedure of Example 24. Deionized water was added to form a 167 mM clear solution. One mole equivalent of sodium hydroxide solution was added to five mole equivalents of the [manganese(II)·(H₂O)₂·(gluconate)₂]⁺²(Cl₂)⁻² solution, giving a ratio of OH⁻ to compound of 0.20. A white precipitate formed instantly and became brown over a period of 10-15 sec, indicating the formation of manganese hydroxide and/or manganese oxides with loss of paramagnetism.

## EXAMPLE 24

$$[Manganese(II)\cdot (H_2O)_2\cdot (gluconate)_2]^{+2}(Cl_2)^{-2}$$

A non-chelate coordination compound solution of [manganese(II)$\cdot$(H$_2$O)$_2$$\cdot$(gluconate)$_2$]$^{+2}$(Cl$_2$)$^{-2}$ was formed in aqueous media at room temperature. Manganese chloride (Baker analytical grade 99.5%; 1.15 g; 5.81 mmole) was dissolved in 11.62 ml of water to form a 500 m$M$ solution. Sodium $D$-gluconate (1,2,3,4,5-pentahydroxycaproic acid; Sigma Chemical Co., St. Louis, MO; 98% purity, lot #114F-0519; anhydrous MW 218.1; 1.09 g; 5 mmole) was dissolved in 10 ml of water to form a 500 m$M$ solution. One part by volume of the manganese chloride solution was added to two parts of the sodium gluconate solution to form, instantaneously, a non-chelate coordination compound solution of [manganese(II)$\cdot$(H$_2$O)$_2$$\cdot$(gluconate)$_2$]$^{+2}$-(Cl$_2$)$^{-2}$. The solution was clear, uncolored and had a pH of 4.8.

## EXAMPLE 25

$$Safety\ Factor\ of\ [Manganese(II)\cdot (H_2O)_2\cdot (glycine)_2]^{+2}(Cl_2)^{-2}$$

NMRI compounds must combine superior safety and efficacy to be useful pharmaceutical drugs. One measure of the safety factor is the numerical ratio of the dose which kills 50% of a test population of animals (LD$_{50}$) and the dose which is needed to achieve a certain level of relaxation enhancement in blood or plasma, for example, the dose which is needed to lower the blood's normal T$_1$ relaxation value from 1500 to 200 msec at 0.15 T in the Spin Analyzer (RADX, Houston, TX).

These data were determined and are shown below in Table A.

TABLE A

| Efficacy, Toxicity and Safety Factor for Manganese Compounds | | | |
|---|---|---|---|
| Compound | LD$_{50}$ | Plasma Dose, T$_1$ = 200 | Safety Factor |
| MnCl$_2$$\cdot$4H$_2$O | 0.1 | 0.01 | 10 |
| MnEDTA | 5.0 | 0.13 | 38 |
| Mn(acetate)$_2$-$bis$-glycine | 0.7 | 0.10 | 70 |

## EXAMPLE 26

$$NMRI\ Efficacy\ of\ [Manganese(II)\cdot (H_2O)_2\cdot (glycine)_2]^{+2}(Cl_2)^{-2}$$

*Relaxation Enhancement in Heart and Liver*

[Manganese(II)$\cdot$(H$_2$O)$_2$$\cdot$(glycine)$_2$]$^{+2}$(Cl$_2$)$^{-2}$ was prepared at 161 m$M$ according to the method of Example 3. This solution was infused into a New Zealand white rabbit (3.1 kg wt.) at 0.065 mmole/kg/min for 5 min to achieve a total dose of 0.31 mole/kg. The rabbit tolerated the infusion without outward signs of a pharmacologic or toxicologic effect. 15 Min after the infusion, the rabbit was sacrificed by lethal pentobarbitol injection, and the heart and liver were removed. Samples of these organs were tested for relaxation time at 0.15 T with a RADX spectrometer (Houston, TX). The liver tissue under normal conditions without any injections has a $T_1$ of 289 +/- 14 msec and a $T_2$ of 50 +/- 5 msec. After this [manganese(II)$\cdot$($H_2O$)$_2\cdot$(glycine)$_2$]$^{+2}$($Cl_2$)$^{-2}$, the liver $T_1$ was 19 msec, and the liver $T_2$ was 10 msec. The heart tissue under normal conditions has a $T_1$ of 504 +/- 37 msec. Under the conditions of this test, the heart had a $T_1$ of 45 msec and a $T_2$ of 21 msec. These values are the lowest relaxation times for liver and heart tissue heretofore reported.

At a lower dose level (0.10 mmole/kg), the effects of a rapid bolus injection (10 sec) or a slow infusion (over 5 min) were tested in two New Zealand white rabbits. 15 Min after the end of the infusion or injection, the animals were sacrificed and $T_1$ and $T_2$ values were measured on 4-16 individual samples of kidney, liver and heart tissue. The results are shown in Table B.

## TABLE B

| Organ | Normal | Tissues | Observed Values | | | |
| | | | Bolus Injection | | Infusion | |
| | $T_1$ | $T_2$ | $T_1$ | $T_2$ | $T_1$ | $T_2$ |
|---|---|---|---|---|---|---|
| Kidney | 488 | 82 | 96 | 39 | 109 | 39 |
| | 656 | 119 | 121 | 43 | 122 | 43 |
| | 381 | 71 | 104 | 34 | 70 | 40 |
| | 500 | 88 | 109 | 43 | 128 | 43 |
| | 481 | 88 | 106 | 36 | 126 | 40 |
| | 544 | 90 | 98 | 35 | 131 | 42 |
| | - | - | 109 | 40 | 114 | 43 |
| | - | - | 116 | 43 | 113 | 45 |
| | - | - | 103 | 38 | 105 | 44 |
| | - | - | 95 | 41 | 143 | 57 |
| | - | - | 115 | 36 | 129 | 46 |
| | - | - | 109 | 39 | 152 | 55 |
| | - | - | 131 | 54 | - | - |
| | - | - | 108 | 36 | - | - |
| Kidney | - | - | 116 | 38 | - | - |
| | - | - | 104 | 42 | - | - |
| Mean | 508 | 90 | 109 | 40 | 120 | 45 |
| Std.Dev. | 90 | 16 | 9 | 5 | 21 | 6 |

TABLE B (contd)

| Organ | Normal | Tissues | Observed Values | | | |
|---|---|---|---|---|---|---|
| | | | Bolus Injection | | Infusion | |
| | $T_1$ | $T_2$ | $T_1$ | $T_2$ | $T_1$ | $T_2$ |
| Liver | 288 | 49 | 46 | 16 | 33 | 15 |
| | 275 | 45 | 54 | 18 | 30 | 11 |
| | 273 | 46 | 55 | 20 | 41 | 15 |
| | 300 | 53 | 52 | 19 | 50 | 20 |
| | 309 | 58 | 49 | 16 | - | - |
| | 286 | 48 | 51 | 17 | - | - |
| Mean | 289 | 50 | 51 | 18 | 39 | 15 |
| Std.Dev. | 14 | 5 | 3 | 2 | 9 | 4 |
| Heart | - | - | 164 | 42 | 164 | 47 |
| | - | - | 166 | 36 | 194 | 42 |
| | - | - | 141 | 45 | 178 | 43 |
| | - | - | 153 | 37 | 180 | 32 |
| Mean | - | - | 156 | 40 | 179 | 41 |
| Std.Dev. | - | - | 12 | 4 | 12 | 6 |

As shown in Table B, the kidney $T_1$ value changed from 504 msec to 109 or 120 msec for bolus and infusion techniques, the bolus injection giving the lower value. On the other hand, the infusion technique lowered the liver $T_1$ to 39 (from a 289 normal value) while the bolus method gave a $T_1$ of 51 msec. In the heart, the bolus method was superior to the infusion method for lowering the tissue $T_1$. Thus, the rate of infusion changes the organ distribution of these agents, a phenomenon that has not been observed from manganese salts, manganese chelates, or other kinds of paramagnetic chelates, or paramagnetic organic stable free radicals, like nitroxides.

## EXAMPLE 27

*NMR Imaging with Manganese(II)$\cdot$ (H$_2$O)$_2$ $\cdot$ (glycine)$_2$]$^{+2}$(Cl$_2$)$^{-2}$*

Magnetic resonance imaging of the compound of Example 3 was performed under clinically useful conditions. A General Electric Signa Magnetic Resonance Imaging System, operating at 1.5 T, and using a knee surface coil was used. New Zealand white rabbits (ca. 2.5 kg wt.) were used for imaging. Doses of 10 and 20 $\mu$mole/kg were studied using a Spin Echo pulse sequence (TR = 300 msec; TE = 20 msec; 5 mm slice thickness; 2.5 mm skip between slices; 2 averages; 128 x 256 image matrix). With these instrument settings, an image could be acquired in about 1.3 min. Images of the rabbit kidney and liver were taken before injection and at 1, 3, 6, 8, 12, 16, 21, 26, 36, 46, and 56 min intervals after injection.

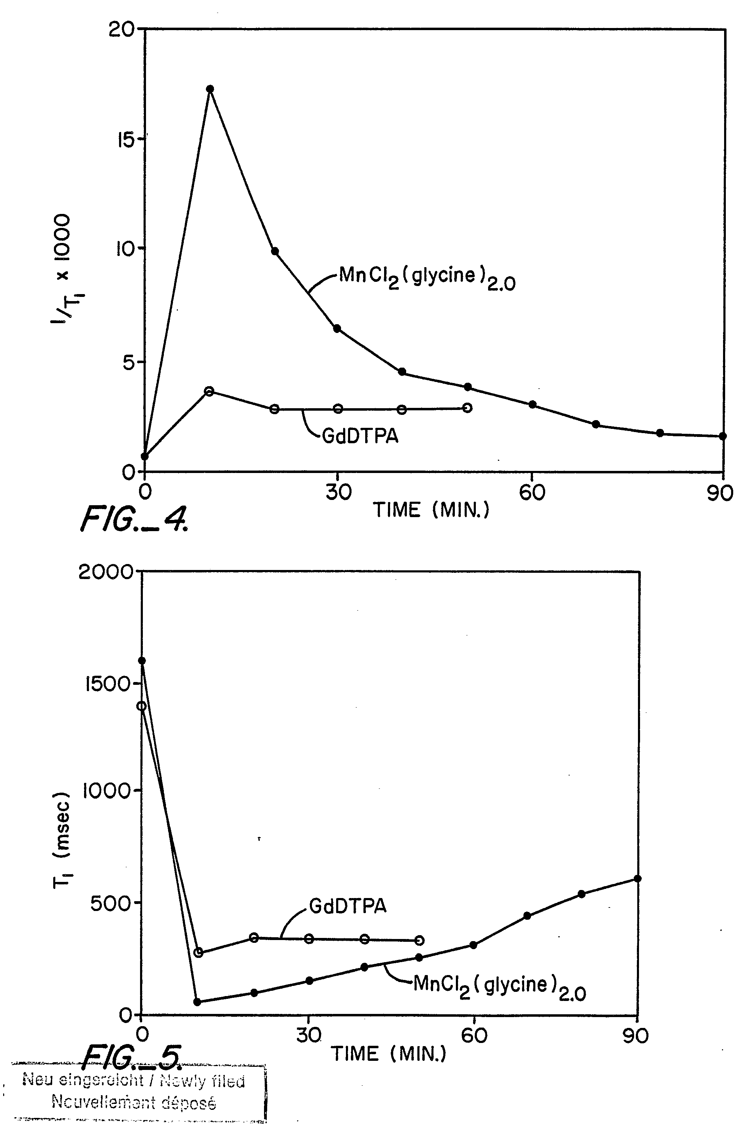

Schematic drawings of the images before injection and at intervals of 10 and 60 minutes are show in Fig. 1, 2 and 3, respectively. The hepatic image before contrast medium injection (Fig. 1) showed good detail, with the gall bladder 2 appearing as a light area against the darker liver parenchyma 6. The intrahepatic vessels 4 are clearly visible as dark areas against the lighter liver parenchyma 6. The vertebra 8 and paraspinal muscles 10 are also visible. However, no functional information was obtained. At one min after injection of Manganese(II)$\cdot$(H$_2$O)$_2$ $\cdot$(glycine)$_2$]$^{+2}$(Cl$_2$)$^{-2}$, the liver and parenchyma were enhanced over 100%.

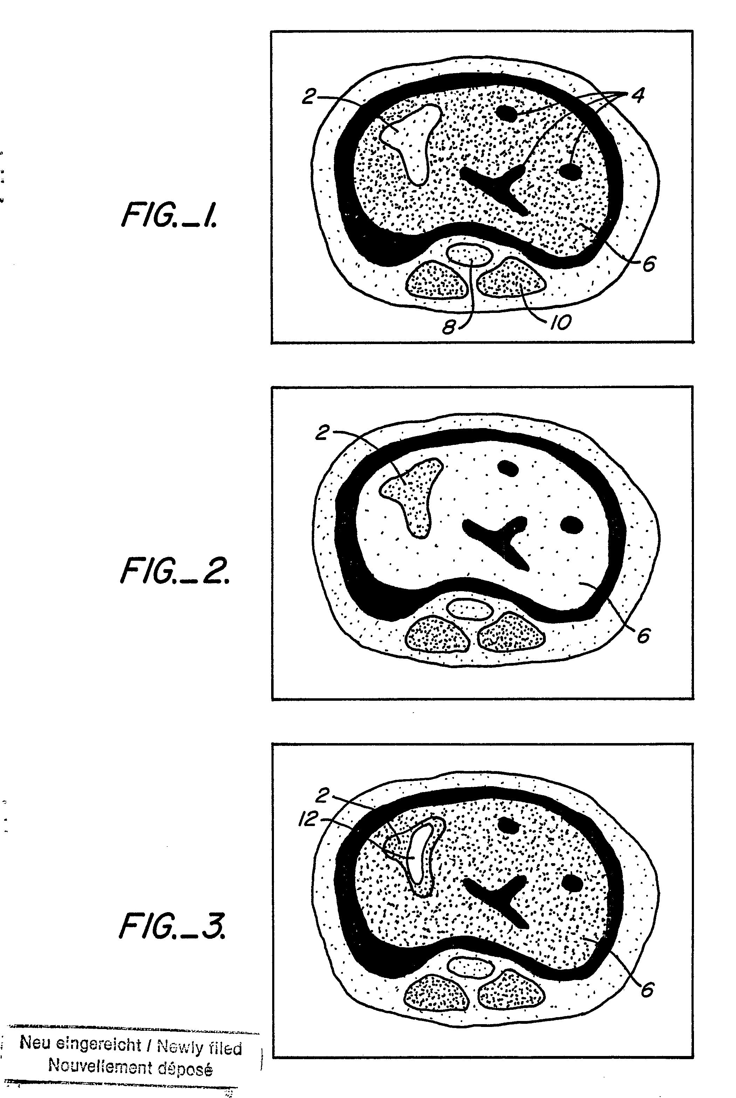

The image after 10 min is shown in Fig. 2. The liver parenchyma 4. From one to 16 min post injection, the liver parenchyma now appears as a light area, lighter than the gall bladder 2, showing concentration of relaxivity in the liver parenchyma. The gall bladder now appears as a dark area against the background of the lighter liver parenchyma.

The image after one hour (60 min) is shown in Fig. 3. The liver parenchyma 6 had returned to normal intensity, but an area 12 of the gall bladder 2 was brighter than at anytime during the study, indicating the concentration of the paramagnetic manganese compound in the gall bladder. It is well know that manganese compounds are first transported from the plasma to the liver parenchyma and then into the bile cannicula and ultimately into the gall bladder. This example shows that the Manganese(II)$^{\bullet}$(H$_2$O)$_2$$^{\bullet}$(glycine)$_2$]$^{+2}$(Cl$_2$)$^{-2}$ also obeys the normal pathway for manganese transit. More importantly, this example also demonstrates that the non-chelated manganese(II) coordination compounds with amino acids remains strongly paramagnetic and thus provides excellent imaging of a biochemical, functionally important process by magnetic resonance.

## EXAMPLE 28

### Plasma Clearance of MRI Compounds

To be useful for imaging the perfusion of blood through organs or to visualize a disruption in the blood brain barrier, a NMRI compound must lower the $T_1$ of blood or plasma substantially and for a time period which is clinically convenient. If the relaxation enhancement effect is short lived, enough time may not be available to inject the patient and then place him or her in the machine for imaging.

Since a dose of 0.1 mmole/kg of GdDTPA has been shown to be useful in extensive human studies, this dose was selected as a standard for evaluating plasma relaxation in rabbits with other compounds.

A dose of 0.1 mmole/kg of GdDTPA was injected into a rabbit and blood samples were drawn at 10 min intervals for 50 min. The blood was centrifuged, and the plasma $T_1$ and $T_2$ times were measured at 0.15 T with a RADX PROTON SPIN ANALYZER (Houston, TX). The procedure was repeated with 0.1 mmole/kg of Manganese(II)$^{\bullet}$(H$_2$O)$_2$$^{\bullet}$(glycine)$_2$]$^{+2}$(Cl$_2$)$^{-2}$, the blood samples being drawn at 10 min intervals for 90 min.

The data obtained are shown in Table C, and a plot of the data (1/$T_1$ x 1000)is shown in Fig. 4.

TABLE C

| (min) | MnGlycine, 0.1 mmole | | GdDTPA, 0.1 mmole | |
|---|---|---|---|---|
| | $T_1$ (msec) | $T_2$ (msec) | $T_1$ (msec) | $T_2$ (msec) |
| 0 | 1600 | 447 | 1380 | 588 |
| 10 | 58 | 42 | 269 | 245 |
| 20 | 102 | 65 | 349 | 287 |
| 30 | 156 | 110 | 348 | 283 |
| 40 | 224 | 156 | 352 | 270 |
| 50 | 259 | 180 | 340 | 298 |
| 60 | 323 | 205 | - | - |
| 70 | 453 | 257 | - | - |
| 80 | 550 | 293 | - | - |
| 90 | 625 | 295 | - | - |

As can be seen in Table C, GdDTPA lowered plasma $T_1$ from 1380 to 259 msec at 10 min. The same dose of Manganese(II)$^{\bullet}$(H$_2$O)$_2$$^{\bullet}$(glycine)$_2$]$^{+2}$(Cl$_2$)$^{-2}$ lowered plasma $T_1$ from 1600 to 58 msec and maintained the plasma $T_1$ below 250 for 40 min. GdDTPA was effective for maintaining the plasma $T_1$ below 300 for only 10 min.

The graphical plot of 1/$T_1$ x 1000 vs time is shown in Fig. 4, showing the extended time for clinical usefulness of Manganese(II)$^{\bullet}$(H$_2$O)$_2$$^{\bullet}$(glycine)$_2$]$^{+2}$(Cl$_2$)$^{-2}$. The graphical plot of $T_1$ vs time is shown in Fig. 5, showing the improved relaxivity obtained with Manganese(II)$^{\bullet}$(H$_2$O)$_2$$^{\bullet}$(glycine)$_2$]$^{+2}$(Cl$_2$)$^{-2}$.

## EXAMPLE 29

*Toxicity of Manganese Salt and Coordination Compounds*

Manganese coordination compounds were formulated by the procedure of Example 3 but with a range of molar ratios of glycine to manganese. These formulations and a manganese chloride solution were tested for acute toxicity by intravenous injection (using the tail vein) into groups of 4 to 6 white mice. The mice were observed for toxicity for up to 4 days, and the $LD_{50}$ dose calculated by standard methods. The results are shown in table D.

TABLE D

| Compound | $LD_{50}$ (mmole/kg) |
|---|---|
| $MnCl_2$ | 100 |
| $Mn(acetate)_2$ | 130 |
| $MnCl_2(glycine)_{1.0}$ | 264 |
| $MnCl_2(glycine)_{2.0}$ | 765 |
| $MnCl_2(glycine)_{2.5}$ | 400 |
| $MnCl_2(glycine)_{5.0}$ | 300 |
| $MnCl_2(glycine)_{6.0}$ | 375 |

## EXAMPLE 30

*Other [Manganese(II)-(H$_{2O}$)$_2$(Amino Acid)$_2$](Dianion) Coordination Compounds*

Repeating the procedure of Example 21, but replacing Mn(II) dichloride with an equimolar amount of the following manganese salts yields a solution of the corresponding [manganese(II)-(H$_2$O)$_2$(amino acid)$_2$]-(dianion) compound which yields greatly enhanced relaxivity in NMR imaging: manganese dibenzoate, manganese diboride, manganese dibromide, manganese dicarbide, manganese dicarbonate, manganese dichloroplatinate, manganese dichromite, manganese dicitrate, manganese ferrocyanide, manganese fluogallate, manganese fluosilicate, manganese fluoride, manganese difluoride, manganese trifluoride, manganese formate, manganese(II) glycerophosphate, manganese hydroxide, manganese diiodide, manganese diiodide tetrahydrate, manganese hexaiodoplatinate, manganese(II) nitrate, manganese(II) lactate, manganese(II)oxalate, manganese(II) oxalate dihydrate, manganese(II) oxalate trihydrate, manganese(II) oxide, manganese dioxide, manganese heptoxide, manganese(II) monoxide, manganese(II) orthophosphate, manganese(II) orthophosphate di-H, manganese(II) orthophosphate mono-H, manganese(II) pyrophosphate, manganese(II) pyrophosphate trihydrate, manganese monophosphide, manganese diphosphide, manganese triphosphide, manganese(II) hypophosphite, manganese(II) orthophosphite, manganese selenate dihydrate, manganese selenate pentahydrate, manganese selenide, manganese selenite, manganese(II) metaslicate, manganese disilicide, manganese monosilicide, manganese(disilicide), manganese(II) sulfate, manganese(II) sulfate dihydrate, manganese(II) sulfate heptahydrate, manganese(II) sulfate hexahydrate, manganese(II) sulfate monohydrate, manganese(II) sulfate pentahydrate, manganese(II) sulfate tetrahydrate, manganese(II) sulfate trihydrate, manganese(II) sulfide, manganese(IV) sulfide, manganese(II) tantalate, manganese(II) tartrate, manganese(II) thiocyanate, manganese(II) dithionate, manganese(II) titanate, manganese valerate, manganese permanganic acid, and manganese manganocyanic acid.

## EXAMPLE 31

*[Manganese(II)H₂O(L-Alanine)₃](Acetate Iodide)*

The title compound was formed in aqueous media at room temperature. Manganese(II) acetate tetrahydrate (Aldrich Chemical Co.; 99 + % purity: lot no. 01730MM 245.09) was put into solution by weighing out 4.127 parts by weight and adding it to 20 parts water by volume to form a 841.9 m$M$ solution. Manganese(II) iodide tetrahydrte (Alpha Products, Danvers, MA; lot no. 092382) was put into solution by weighing out 3.628 parts by weight and adding it to 570 parts by volume to form a 16.7 m$M$ solution. *L*-Alanine (α-aminopropionic acid; Pierce Chemical Co., Rockford, IL; MW 89.09) was put into solution by weighing 0.9233 parts of alanine by weight and adding it to 10 parts by weight water to form a 1.04 $M$ solution.

A 1 m$M$ solution of the title compound was formed by mixing 15 parts by volume on the manganese acetate solution, 744 parts of the manganese iodide solution, and 72 parts of the alanine solution and sufficient water to produce 25,000 parts final solution. The Mn(II)I₂ gave the solution a faint yellow appearance.

The paramagnetic relaxation of the title compound in water was measured at 10 MHz (0.15T) with a spin analyzer (RADX; Houston, TX). After thermal equilibrium, the spin-lattice ($T_1$) and spin-spin ($T_2$) relaxation values were measured. The $T_1$ value of the 1 m$M$ solution was 104 msec, and the $T_2$ value was 26 msec. These values permitted the calculation of the relaxivity of 9.62 m$M^{-1}sec^{-1}$ for $T_1$ and 38.5 m$M_{-1}sec_{-1}$ for $T_2$.

## EXAMPLE 32

*[Manganese(II)·Glycine·L-Serine·L-Histidine·L-Proline](Sulfate)*

The title compound was formed in aqueous media at room temperature. Manganese sulfate monohydrate (EM Scientific; lot no. 6069; 98% pure, MW 169.01) was put into solution by weighing out 1.9055 parts by weight into 20 parts water by volume to give a clear solution of 564 mM. The indicated amino acids (Pierce Chemical Co.) were each put into separate aqueous solutions: 2.2824 parts glycine by weight and 20 parts water by volume gave a 1.52 $M$ solution; 0.6465 parts serine and 10 parts water by volume gave a 615 m$M$ solution; 1.4077 parts histidine by weight and 130 parts water by volume gave a 69.8 m$M$ solution; 0.3477 parts proline by weight and 10 parts water by volume gave a 302 m$M$ solution.

A 1 m$M$ solution of the title compound was formed by mixing (by volume): 44 parts MnSO₄, 16 parts glycine, 41 parts serine, 348 parts histine, and 83 parts proline with sufficient water to form 25,000 parts final solution.

The $T_1$ and $T_2$ relaxivity of the title compound was determined as specified in Example 1. The $T_1$ of the 1 m$M$ solution was 103 msec and the $T_2$ was 28 msec. These values give a relaxivity of 9.71 m$M^{-1}sec^{-1}$ for $T_1$ and 35.7 m$M^{-1}sec^{-1}$ for $T_2$.

## EXAMPLE 33

*[Manganese(II)(H₂O)₂(L-serine)₂](Glycerophosphate)*

The title compound was formed in aqueous solution at room temperature. Manganese glycerophosphate (Spectrum Chemical Co., Gardena, CA; lot no. CH263; 98% purity; 12% loss on drying; MW 225) was put into solution by adding 0.3348 parts manganese glycerophosphate by weight to 520 parts water by volume and heating to 54°C for 1 hour. The serine solution of Example 32 was used herein.

The title compound was obtained by mixing 8,740 parts by volume of manganese glycerophosphate with 81 parts of the serine solution with sufficient water to form 25.000 parts by volume of a 1 mM solution.

The $T_1$ and $T_2$ of the title compound were determined as specified in Example 5. The $T_1$ of the 1 mM above formed solution was 147 msec and the $T_2$ was 54 msec. The relaxivity was 6.80 m$M^{-1}$sec$^{-1}$ for $T_1$ and 18.5 m$M^{-1}$sec$^{-1}$ for $T_2$. These values are lower than typically seen for compositions of this invention.

## EXAMPLE 34

### [Manganese(II)(H₂O)₃-L-Serine]Digluconate

The title compound was formed in aqueous solution at room temperature. Manganese gluconate dihydrate (Spectrum Chemical, Gardena, CA; lot no. BB316; 98% pure; FW 481.27) was put into solution by weighing out 2.8759 parts by weight into 130 parts water by volume to form a 45.97 mM solution. The serine solution of Example 32 was used herein.

The title compound was obtained by mixing 543 parts by volume of manganese digluconate solution with 41 parts by volume of the serine solution with sufficient water to form 25,000 parts by volume of a 1 mM solution.

The $T_1$ and $T_2$ relaxivity of the title compound was determined as specified in Example 1. The $T_1$ of the 1 mM solution above formed was 107 msec and the $T_2$ was 27 msec. The relaxivity was 9.35 m$M^{-1}$sec$^{-1}$ for $T_1$ and 37.0 m$M^{-1}$sec$^{-1}$ for $T_2$.

## EXAMPLE 35

### Comparative Relaxivity of [Manganese(II)•(H₂)ₘ•(Glycine)ₙ](Diacetate) where (m + n) = 4 and n = 0, 1, 2, 3, 4.

Since Mn(II) coordination compounds are hexadentate, as many as four zwitterionic amino acids can occupy inner coordination positions, as well as the two positions occupied by the necessary anions. With each additional amino acid in the coordination compound, one less water can be present in the inner coordination sphere. These experiments were conducted to determine if relaxivity was adversely affected by the exclusion of inner sphere water in the homologous series of title compounds. The manganese diacetate solution of Example 31 and the glycine solution of Example 32 were used.

Precise and accurate dilutions were essential for this experiment. A 5 mM solution of manganese acetate and glycine were each made: 60 parts of the manganese acetate solution of Example 31 and 9,940 parts water to form 10,000 parts of a 5 mM solution; 82 parts of the glycine solution of Example 32 and 24,920 parts water to form 25,002 parts of a 5 mM solution.

The solutions denoted in Table E below were formed by mixing 1 part 5 mM Mnacetate and X parts 5 mM glycine with (4 - X) parts water to form 5 parts of a 1 mM solution, where X is the subscript "n" in the compound name.

A solution with 336 mole equivalents of glycine was also formed by replacing 1 part of water with 1 part by volume of the 1.52 M glycine solution of Example 32.

Relaxivity was measured at 10 MHz (0.15T) as described in Example 10.

TABLE E

| Compound | Relaxivity (m$M^{-1}$sec$^{-1}$) | |
| --- | --- | --- |
| | Spin-Lattice | Spin-Spin |
| [Manganese(II)$\cdot$(H$_2$O)$_4$]Diacetate | 9.09 | 33.3 |
| [Manganese(II)$\cdot$(H$_2$O)$_3$]$\cdot$(Glycine)]Diacetate | 8.26 | 33.3 |
| [Manganese(II)$\cdot$(H$_2$O)$_2$$\cdot$(Glycine)$_2$]Diacetate | 8.40 | 34.5 |
| [Manganese(II)$\cdot$(H$_2$O)$\cdot$(Glycine)$_3$]Diacetate | 8.47 | 33.3 |
| [Manganese(II)$\cdot$(Glycine)$_4$]Diacetate | 8.00 | 33.3 |
| [Manganese(II)$\cdot$(Glycine)$_4$]Diacetate + Glycine 336 | 7.81 | 25.6 |

The data are consistent with the theory that with each additional glycine within the inner coordination sphere, there is a loss in relaxivity because water is excluded. The reason the relaxivity losses are small is because the exchange reaction of water and glycine occurs very rapidly on the time scale of the NMR measurement process.


**EXAMPLE 36**


*Enhancement of Stability of [Manganese(II)$\cdot$(H$_2$O)$_2$$\cdot$(Glycine)$_2$](Diacetate) Solutions with Buffers*


The solution stability of the title compound can be enhanced by buffering the pH of the solution. Three buffers were tested for suitability.

One part of the solution of the title compound, synthesized according to Example 35, was added to one part of a US National Bureau of Standard buffer solution for pH 4.0 (American Scientific Products; Phthalate reference buffer). The solution remained clear, the pH was 4.69, and the relaxivity did not change over a 12 hour time period.

In a similar manner, one part of the title compound was mixed with one part of a phosphate pH reference buffer (American Scientific Products; pH 7.0). The solution remained clear, the pH was 7.63, and the relaxivity did not change over a 12 hour time period.

In a similar manner, one part of the title compound was added to one part of a carbonate pH reference buffer (American Scientific Products; pH 10.0). The solution remained clear, the pH was 10.45, and the relaxivity did not change over a 12 hour time period.

Other buffer systems that are found to be suitable include the 26 "Buffer Solutions" described on pp. J-234 - J-237 of the Handbook of Biochemistry (Supra), the "New Buffers for Biological Research" described on page J-238 of the HANDBOOK OF BIOCHENISTRY (supra), and the Buffering Agents described on page 1491 of the U.S. PHARMACOPEIA, XXI Edition, the entire contents of which and the references cited therein being hereby incorporated by reference in their entireties.


**EXAMPLE 37**


*Toxicity of Mn(II) Salt and Coordination Compound Formulations Containing Calcium Salts*


Calcium salts have been shown to compete with Mn salts for certain biological targets, especially in heart tissue. These experiments were conducted to test if calcium salts could improve the safety of the manganese coordination compounds of this invention.

All compounds and formulations were made according to the methods of Example 1. Calcium salts were added as indicated in the Table below, as the solid to preformed Mn solutions. The Ca salts were dissolved, the final solutions filtered through a 0.22 filter to remove debris, and the toxicity tested under standard methods in mice. The LD50 was estimated using conventional methods.

TABLE F

| Coordination Compound | Calcium Addition | $LD_{50}$ mmoles/kg |
|---|---|---|
| [Manganese(II) $\cdot$ (H$_2$O) $\cdot$ (Histidine)$_2$](diacetate) | None | 300 |
| CaCl$_2$ | 1600 | |
| [Manganese(II) $\cdot$ (Glycine)$_4$](dichloride) | 0.25 mol eq CaCl$_2$ | 350 |
| [Manganese(II) $\cdot$ (Glycine)$_4$](dichloride) | 0.50 mol eq CaCl$_2$ | 450 |
| [Manganese(II) $\cdot$ (Glycine)$_4$](dichloride) | 0.75 mol eq CaCl$_2$ | >350 |
| [Manganese(II) $\cdot$ (H$_2$O)$_{1.5}$ $\cdot$ (Glycine)$_{2.5}$](dichloride) | 0.25 mole eq CaCl$_2$ | 400 |
| [Manganese(II) $\cdot$ (H$_2$O)$_2$ $\cdot$ (Glycine)$_2$](diacetate) | 0.4 mole eq Ca Diacetate | 300 |
| [Manganese(II) $\cdot$ (Glycine)$_4$](diacetate) | 0.6 mole eq Ca Diacetate | 375 |

These results support the thesis that calcium salts may increase the safety of manganese coordination compounds, although the effects are small and variable.

## EXAMPLE 38

$$[Manganese(II) \cdot CH_2(NH_2)COO\text{-}]_2^+ {}^{'}(SO_4^=)$$

The title compound was synthesized according to the methods of Example 2, U.S. Patent No. 3,950,372, by Mahmoud M. Abdel-Monem. 1.5 G Glycine (Baker Grade: Lot 603713; purity 99.8%) was dissolved by stirring in 10 mL distilled water. Manganese sulfate monohydrate, (EM Scientific; Lot 6069) 3.3 g, was dissolved over 15 min. in 25 ml distilled water with stirring and heat (50° C). Both solutions were mixed together and further heated for one hr at 70° C under aspirator vacuum. The pink clear viscous solution (reduced in volume to about 30 ml) was cooled and added to 150 ml absolute ethanol, to form a turbid white suspension. Upon standing, the turbid layer separated. Over time, a white crystalline solid formed at the bottom of the flask. This was filtered, separated, powdered, washed twice with absolute ethanol, and once with acetone and dried under vacuum.

The material decomposed without a well defined melting point. Assuming the formula weight for [H$_2$C-(H$_2$N)COO$^-$ $\cdot$ Mn(II)]$_2^+$ (SO$_4^=$) of 352, 0.4648 gram (1.32 mmole) was weighed out and added to 100 ml to give a 13.2 m$M$ solution.

The relaxivity of the title compound was performed at 0.15 T (10 MHz) with a RADX Spin Analyzer (Houston, TX) at 37° C. Table G below compares (a) a manganese salt product of Example 35, (b) manganese coordination compound of this invention made according to the procedure of Example 35, and (c) a 1:1 manganese $\alpha$-amino acid complex synthesized according to U.S. Patent 3,950,372. The manganese coordination compounds of this invention had the surprisingly large and useful relaxivity of Mn salts, while a *greatly* reduced relaxivity is seen for the manganese amino acid chelates of U.S. Patent 3,950,372.

26

TABLE G

| Compound | Spin-Spin Relaxivity $(mM^{-1}sec^{-1})$ |
|---|---|
| (a) $[Manganese(II) \cdot (H_2O)_4]^{+2}(Acetate)^{-2}$ | 33.30 |
| (b) $[Manganese(II) \cdot (H_2O)_3 \cdot (CH_2(NH_3)^+COO^-)_1]^{+2}(Acetate)^{-2}$ | 33.30 |
| (c) $[Manganese(II) \cdot (H_2O)_3 \cdot (CH_2(NH_2)COO^-)_1]_2^{+1}(SO_4^=)^{-2}$ | 2.92 |

**Claims**

1. A method for performing an NMR diagnostic procedure comprising administering a physiologically acceptable solution of an NMR image enhancing amount of a non-chelate coordination compound of the formula:

$$(Mn(II)(H_2O)_m A1_n A2_o A3_p A4_q)^{+a}(Y, Z_r)^{-a}$$

wherein:
A1, A2, A3 and A4 are the same or different amino-substituted carboxylic acid groups having from 2 to 18 carbons;
Y and Z are each the same or a different anion of a pharmaceutically acceptable inorganic acid or an organic carboxylic acid having from 2 to 18 carbons;
a is valence of the ions;
m, n, o, p and q are each 1 or 0, (m + n + o + p + q) = 4; and r is 1, and if Y is a multivalent anion, r is 0 or 1.

2. The method of claim 1, wherein (n + o + p + q) is from 1 to 3.

3. The method of claim 1, wherein (n + o + p + q) is from 2 to 4.

4. The method of any one of claims 1 to 3, wherein Y and Z are monovalent.

5. The method of claim 4, wherein Y is an aliphatic carboxylic acid anion having from 2 to 10, preferably 2 to 6 carbons and Z is chloride.

6. The method of claim 4, wherein Y is an anion of acetic, propionic, n-butyric, isobutyric, valeric, isovaleric, gluconic, lactic or pivalic acid.

7. The method of any one of claims 1 to 6, wherein A1, A2, A3 and A4 are $\alpha$-amino acids, preferably having from 2 to 10 carbons, such as glycine.

8. The method of any one of claims 1 to 7, wherein the solution has a pH of from 4.0 to 9.5.

9. The method of imaging body tissue in a patient comprising subjecting the patient to NMR tomography, which method comprises, prior to performing the NMR tomography, administering to the patient an effective amount of a solution of a pharmaceutical agent for affecting the relaxation times of atoms in body tissues undergoing NMR diagnosis, whereby the image is enhanced, said agent comprising an amount, effective to affect such relaxation times, of a compound of the formula:

$$(Mn(II)(H_2O)_m A1_n A2_o A3_p A4_q)^{+a}(Y, Z_r)^{-a}$$

wherein A1, A2, A3, A4, X, Z, a, m, n, o, p, q and r are as defined in any one of claims 1 to 8.

10. The method of claim 9, wherein the body tissue undergoing NMR diagnosis is heart or liver.

11. The method of claim 9 or 10, wherein the pH of the solution is within the range of from 4.0 to 9.5.

12. An NMR image enhancing composition comprising a physiologically acceptable parenteral solution containing an image enhancing concentration of a compound of the formula:

$$(Mn(II)(H_2O)_m A1_n A2_o A3_p A4_q)^{+a}(Y, Z_r)^{-a}$$

wherein A1, A2, A3, A4, Y, Z, a, m, n, o, p, q and r are as defined in any one of claims 1 to 8.

13. The NMR image enhancing composition of claim 12, wherein n is from 1.5 to 4, preferably 2 to 3.

14. The NMR image enhancing composition of claim 12 or 13, wherein the solution pH is from 4 to 9.5, preferably 5 to 7.

15. The NMR image enhancing composition of any one of claims 12 to 14, wherein the compound concentration is from 0.5 to 4.0 weight percent.

FIG._4.

FIG._5.

FIG._1.

FIG._2.

FIG._3.